# FASCICULE DE BREVET EUROPEEN

(11) **EP 3 404 106 B1**
(45) Date de publication et mention de la délivrance du brevet: **09.06.2021**
(21) Numéro de dépôt: 18182802.1
(22) Date de dépôt: 10.04.2014
(51) Int. Cl.: C12N 15/86, C07K 14/47, C12N 15/113, A61K 48/00, C12N 9/16, C12N 9/64, A61K 38/46, A61K 38/48, A61P 21/00, A61P 25/00, A61P 43/00

(54) **SYSTEME D'EXPRESSION POUR UNE THERAPIE GENIQUE SELECTIVE**
SELEKTIVES GENTHERAPIEEXPRESSIONSSYSTEM
SELECTIVE GENE THERAPY EXPRESSION SYSTEM

(30) Priorité: 11.04.2013 FR 1353306
(43) Date de publication de la demande: 21.11.2018
(62) Demande divisionnaire de: 14721468.8
(73) Titulaire: GENETHON, 91002 Evry Cedex (FR); Centre National de la Recherche Scientifique, 75794 Paris (FR)
(72) Inventeur: BUJ BELLO, Ana Maria, 75005 PARIS (FR); RICHARD, Isabelle, 91100 CORBEIL ESSONNES (FR)
(74) Mandataire: Cabinet Laurent & Charras

(56) Documents cités:
- CN-A- 102 980 920
- C Karine ET AL: "Toxicity of uncontrolled activity of calpain 3" In: "Myology 2011, 4th International Congress of Myology", 2011, XP055126693, page 119, * abrégé *
- XIE JUN ET AL: "MicroRNA-regulated, Systemically Delivered rAAV9: A Step Closer to CNS-restricted Transgene Expression", MOLECULAR THERAPY, NATURE PUBLISHING GROUP, GB, vol. 19, no. 3, mars 2011 (2011-03), pages 526-535, XP009148361, ISSN: 1525-0016
- ELIZABETH J KELLY ET AL: "MicroRNAs and the Regulation of Vector Tropism", MOLECULAR THERAPY, vol. 17, no. 3, mars 2009 (2009-03), pages 409-416, XP055007015, ISSN: 1525-0016, DOI: 10.1038/mt.2008.288
- WU ET AL: "Adeno-associated Virus Serotypes: Vector Toolkit for Human Gene Therapy", MOLECULAR THERAPY : THE JOURNAL OF THE AMERICAN SOCIETY OF GENE THERAPY, ACADEMIC PRESS ; NATURE PUBLISHING GROUP, US, vol. 14, no. 3, 12 août 2006 (2006-08-12), pages 316-327, XP005844824, ISSN: 1525-0016, DOI: 10.1016/J.YMTHE.2006.05.009
- DATABASE UniProt [Online] 1 février 1991 (1991-02-01), "RecName: Full=Calpain-3; EC=3.4.22.54; AltName: Full=Calcium-activated neutral proteinase 3; Short=CANP 3; AltName: Full=Calpain L3; AltName: Full=Calpain p94; AltName: Full=Muscle-specific calcium-activated neutral protease 3;", XP002783512, extrait de EBI accession no. UNIPROT:P20807 Database accession no. P20807
- BARTOLI ET AL: "Safety and Efficacy of AAV-Mediated Calpain 3 Gene Transfer in a Mouse Model of Limb-Girdle Muscular Dystrophy Type 2A", MOLECULAR THERAPY, NATURE PUBLISHING GROUP, GB, vol. 13, no. 2, février 2006 (2006-02), pages 250-259, XP005252884, ISSN: 1525-0016, DOI: 10.1016/J.YMTHE.2005.09.017

## Description

### DOMAINE TECHNIQUE

La présente invention concerne la thérapie génique, en particulier le traitement des maladies affectant les muscles squelettiques.

Dans ce cadre, il est décrit un système d'expression comprenant un transgène codant la calpaine 3, permettant d'assurer la production d'une quantité thérapeutiquement efficace de celle-ci au niveau des muscles squelettiques, et d'une quantité toxiquement acceptable de celle-ci au niveau du cœur.

### ETAT ANTERIEUR DE LA TECHNIQUE

La question du traitement des pathologies musculaires reste cruciale. Le transfert de gène, notamment à l'aide de vecteurs dérivés des virus associés aux adénovirus qui s'avèrent être des outils particulièrement adaptés pour la transfection musculaire, constitue une stratégie particulièrement prometteuse. Il s'agit d'administrer au sujet malade une copie du gène intact, destiné à la production d'une protéine fonctionnelle compensant la protéine mutée et inactive produite par le sujet.

Dans le cas des pathologies musculaires, l'administration peut se faire par injection locale, au niveau musculaire, du vecteur portant le transgène. Toutefois, au niveau clinique, une administration systémique est préférée, ce qui implique que le transgène puisse se retrouver au niveau des différents tissus de l'organisme.

De manière classique, le transgène est placé sous le contrôle de séquences régulatrices gouvernant son expression, notamment concernant le niveau d'expression ou la spécificité tissulaire de l'expression. Ainsi et dans le cas de la thérapie génique d'une maladie musculaire, un promoteur gouvernant une expression plus spécifiquement dans le muscle peut être privilégié. A titre d'exemple, il a été développé un promoteur synthétique C5-12, bien connu de l'homme du métier, censé favoriser l'expression des gènes au niveau des muscles.

Il existe toutefois un besoin évident de développer de nouveaux outils de thérapie génique permettant de traiter les maladies neuromusculaires, conduisant à la production de quantités efficaces de protéine au niveau des tissus cibles permettant de compenser le défaut d'activité de protéines mutées, et qui soient par ailleurs sans danger pour les patients traités.

En relation avec la calpaine 3, Richard I. et al. (Myology 2011, 4th International Congress of Myology, p. 119) ont rapporté la toxicité cardiaque de vecteurs AAV recombinants administrés par voie intraveineuse et ont développé de nouveaux AAV ayant une expression restreinte aux muscles squelettiques, permettant de prévenir cette toxicité cardiaque.

Xie J. et al. (2011, Molecular Therapy, 19(3), p. 526-35) ont rapporté que des vecteurs AAV9 recombinants pouvaient être « déciblés » du cœur en y insérant des séquences cibles d'un miR exprimé dans ce tissu.

### OBJET DE L'INVENTION

La présente demande repose sur la mise en évidence, par les inventeurs, qu'après administration systémique, un système d'expression destiné à la production d'une protéine d'intérêt au niveau d'un tissu cible, avantageusement les muscles squelettiques, peut entraîner de manière simultanée une expression au niveau des autres tissus et organes potentiellement toxique, rendant ledit système inadapté à un usage thérapeutique.

Il est décrit des solutions techniques à ce problème nouvellement identifié, concernant notamment les fuites cardiaques liées à l'expression d'un transgène à visée musculaire squelettique.

Plus largement, il s'agit donc pour un système d'expression donné :
- de déterminer si celui-ci présente une toxicité ;
- de déterminer dans quel(s) tissu(s) celui-ci présente une toxicité ;
- de fournir des moyens pour réduire cette toxicité à un niveau acceptable.

Les protéines visées sont donc celles qui, lorsqu'elles sont exprimées à partir d'un système d'expression, présentent une toxicité dans au moins un tissu, en particulier dans un tissu non cible.

De manière avantageuse, le système d'expression est administré de manière systémique dans l'organisme, en particulier chez un animal, encore plus avantageusement chez l'homme.

De manière préférée, l'analyse de la toxicité est réalisée dans un organisme présentant une copie défectueuse de la séquence codant la protéine d'intérêt, à savoir dans un organisme présentant la pathologie à traiter, par exemple un modèle animal de type « Knockout » (KO). En effet, si dans le cadre de la présente demande, une toxicité cardiaque a pu être observée pour des systèmes d'expression codant la myotubularine ou la calpaine 3, dans le cas de la myotubularine, celle-ci n'a été détectée que dans les souris KO. En d'autres termes, une analyse de toxicité qui, selon la pratique habituelle, aurait été réalisée chez un animal sain, n'aurait pas mis en évidence cette toxicité.

Ainsi et de manière générale, il est décrit un système d'expression comprenant une séquence codant une protéine, ledit système d'expression permettant :
- l'expression à un niveau thérapeutiquement acceptable de la protéine dans le ou les tissus cibles ; et
- l'expression à un niveau toxiquement acceptable de la protéine dans les tissus autres que les tissus cibles, c'est à dire dans les tissus non cibles.

Plus précisément, la présente invention concerne un système d'expression pour administration systémique comprenant une séquence codant la calpaine 3 placée sous le contrôle:
- d'une séquence promotrice permettant l'expression à un niveau thérapeutiquement acceptable de calpaine 3 dans les muscles squelettiques; et
- d'au moins une séquence cible du miR208a.

Le tissu cible est avantageusement défini comme le tissu ou l'organe dans lequel la protéine doit jouer un rôle thérapeutique, notamment dans le cas où le gène natif codant cette protéine est défectueux. Selon l'invention, le tissu cible vise les muscles striés squelettiques, appelés par la suite muscles squelettiques, à savoir l'ensemble des muscles impliqués dans la motricité, y compris le diaphragme. Ces muscles sont notamment affectés dans les pathologies appelés myopathies. Un autre tissu cible potentiellement d'intérêt est le tissu nerveux périphérique, qui peut également être affecté dans les maladies neuromusculaires. Selon l'invention, les tissus cibles comprennent les muscles squelettiques.

Les tissus non cibles sont avantageusement définis comme les tissus ou organes dans lesquels la protéine n'a pas de rôle thérapeutique à jouer, et éventuellement dans lesquels la présence de la protéine en quantité supérieure à la quantité endogène peut s'avérer délétère voire mortelle, et donc toxique.

Des tissus qui sont particulièrement à protéger de cette éventuelle toxicité sont avantageusement :
- le cœur ou muscle strié cardiaque ;
- le foie ;
- le cerveau ;
- les poumons ;
- le rein; et/ou
- les muscles lisses, en particulier le tractus gastro-intestinal.
Il s'agit donc avantageusement d'organes vitaux ou de tissus dans lesquels les systèmes d'expression génique ont tendance à s'accumuler.

Dans le cadre de l'invention, le muscle cardiaque apparaît comme un tissu particulièrement d'intérêt, comme démontré au moins pour la myotubularine et la calpaine 3. Selon l'invention, le système d'expression permet donc une expression à un niveau toxiquement acceptable de la protéine dans le cœur.

Ainsi, il est décrit un système d'expression comprenant une séquence codant une protéine, ledit système d'expression permettant :
- l'expression à un niveau thérapeutiquement acceptable de la protéine dans les tissus cibles comprenant les muscles squelettiques et/ou le tissu nerveux périphérique ; et
- l'expression à un niveau toxiquement acceptable de la protéine dans les tissus autres que les tissus cibles, notamment dans le cœur.

Selon une première caractéristique, le système d'expression comprend une séquence codant une protéine, correspondant à un transgène. Dans le cadre de l'invention, on entend par « transgène » une séquence, avantageusement un cadre de lecture ouvert, apportée en trans à l'aide du système d'expression selon l'invention.

Selon un mode de réalisation particulier, cette séquence constitue une copie, identique ou équivalente, d'une séquence endogène présente au niveau du génome de l'organisme dans lequel est introduit le système d'expression. Selon un mode de réalisation particulier, la séquence endogène présente une ou plusieurs mutations rendant la protéine codée peu ou pas fonctionnelle voire absente (défaut d'expression ou d'activité de la protéine endogène), notamment au niveau des tissus cibles, en particulier des muscles squelettiques. En d'autres termes et de manière avantageuse, le système d'expression selon l'invention est destiné à être administré à un sujet présentant une copie défectueuse de la séquence codant la protéine et présentant une pathologie associée. Dans ce cadre, la protéine codée par la séquence portée par le système d'expression selon l'invention peut donc être définie comme une protéine dont la mutation cause une maladie neuromusculaire.

Selon un autre mode de réalisation, il s'agit d'une séquence codant une protéine capable de « compenser » le défaut d'une protéine défectueuse (au niveau de son expression ou de son activité) chez le sujet auquel est administré le système d'expression. Ainsi et à titre d'exemple en rapport avec les pathologies neuromusculaires :
- l'utrophine peut être utilisée en remplacement d'une dystrophine mutée et déficiente ;
- la décorine, la fibromoduline ou le lumican permettent de compenser la fonte musculaire observée dans le cas de pathologies neuromusculaires ;
- l'activine permet également d'augmenter la masse musculaire dans des pathologies dont la cause n'est pas une mutation de cette protéine.

Ainsi et de manière plus générale, la séquence portée par le système d'expression peut être définie comme codant une protéine présentant une activité thérapeutique dans le cadre d'une maladie neuromusculaire. La notion d'activité thérapeutique est définie comme ci-dessous en relation avec l'expression « niveau thérapeutiquement acceptable ».

La séquence codant la protéine est une séquence d'acide nucléique et peut notamment être un ADN (acide désoxyribonucléique), un ARN (acide ribonucléique) ou un ADNc (acide désoxyribonucléique complémentaire).

De manière avantageuse, ladite séquence code une protéine fonctionnelle, c'est-à-dire capable d'assurer sa fonction native ou essentielle, notamment au niveau du muscle squelettique. Pour chaque protéine d'intérêt, il peut être défini l'activité recherchée et la séquence nécessaire à l'obtention de cette activité.

Selon un mode de réalisation privilégié, ladite séquence code la protéine native, ladite protéine étant avantageusement d'origine humaine. Il peut également s'agir d'un dérivé ou d'un fragment de cette protéine, pourvu que ce dérivé ou ce fragment conserve l'activité recherchée. De manière avantageuse, on entend par « dérivé » ou « fragment » une séquence protéique présentant au moins 60%, préférentiellement 70%, encore plus préférentiellement 80%, voire 90%, 95% ou 99% d'identité avec la séquence humaine de la protéine d'intérêt. Sont ainsi par exemple visées les protéines d'autre origine (mammifères non humains,...) ou des protéines tronquées, voire mutées, mais actives. Ainsi et dans le cadre de l'invention, le terme « protéine » s'entend comme la protéine entière quelque que soit son origine, ainsi que les dérivés et fragments fonctionnels de celle-ci.

Dans le contexte de la présente demande sont notamment visées les protéines permettant la prise en charge thérapeutique de maladies dites neuromusculaires, pouvant affecter les muscles squelettiques et/ou le tissu nerveux périphérique. Sont plus particulièrement visées les protéines permettant la prise en charge thérapeutique de maladies affectant les muscles squelettiques, appelées de manière générique « myopathies ».

Selon un aspect particulier, ces maladies sont causées par des mutations dans au moins un gène entrainant la non production de la protéine ou la production d'une protéine totalement ou partiellement non fonctionnelle. Ainsi, le système d'expression permet de produire cette protéine sous une forme active et dans une quantité permettant de compenser au moins partiellement le défaut de la protéine native, ou une autre protéine capable de compenser le défaut de la protéine native. L'administration du système d'expression permet ainsi d'améliorer voire de restaurer un phénotype normal au niveau du ou des tissus cibles, notamment au niveau des muscles squelettiques en termes de mobilité et de respiration.

Une protéine présentant un intérêt tout particulier dans le cadre de la présente demande est la myotubularine d'origine humaine (SEQ ID NO : 1), murine (SEQ ID NO : 2) ou canine (SEQ ID NO : 3). Toute séquence codant ces protéines, des dérivés ou des fragments fonctionnels au niveau thérapeutique de celles-ci, peut donc être mise en œuvre dans le cadre du système d'expression selon la présente demande.

Ainsi et à titre d'exemple, les séquences nucléotidiques correspondantes (ADNc) sont les séquences SEQ ID NO : 4, 5 (ou 14) et 6, respectivement.

De manière connue, des mutations dans le gène *MTM1* entraînent une maladie musculaire appelée myopathie myotubulaire (MTM ou XLMTM). Ainsi et selon la stratégie de remplacement ou de transfert de gène, l'apport en trans d'une séquence codant une myotubularine thérapeutique, par exemple native, permet de traiter cette pathologie.

Selon l'invention, la protéine d'intérêt est la calpaine 3 (CAPN3) dont des mutations causent notamment une maladie génétique autosomique récessive appelée dystrophie des ceintures de type 2A (LGMD 2A ou calpainopathie, OMIN 253600). A titre d'exemple, la calpaine 3 humaine présente la séquence SEQ ID NO : 7. Ainsi et comme décrit précédemment, toute séquence qui code une calpaine 3 thérapeutique, par exemple celle de séquence SEQ ID NO : 7 ou un dérivé ou fragment de celle-ci, peut être présente dans un système d'expression selon l'invention. Il peut par exemple s'agir de la séquence d'ADNc représenté à la séquence SEQ ID NO : 8 ou les nucléotides 307 à 2772 de celle-ci correspondant au cadre de lecture ouvert.

Une liste non exhaustive de protéines impliquées dans des pathologies affectant les muscles squelettiques est la suivante : Sarcoglycanes (α, β, y, δ), Dystrophine, Dysferline (DYSF), Sélénoprotéine 1 (SEPN1), Amphyphisine 2 (BIN1), dynamien 2 (DNM2), cofiline 2 (CFL2), troponine T (TNNT1), tropomyosine 3 (TPM3), ACTA1, contactine 1 (CNTN1), TRIM32, Rapsyne (RASPN), DOK7, Agrin (AGRN), COLQ, CHAT, récepteurs de l'Acéthylcholine (CHRNE, CHRNA1, CHRNB1, CHRND), GFPT1, MUSK.

Selon un mode de réalisation particulier, la séquence contenue dans le système d'expression ne code pas une protéine de la famille des sarcoglycanes, en particulier l'a-sarcoglycane, plus précisément la séquence décrite dans le document document Mendell et al. (Annals of Neurology, Vol. 68, No 5, pp 629-638, 2010).

Selon un autre mode de réalisation particulier, la séquence contenue dans le système d'expression ne code pas une protéine de type dystrophine, notamment une minidystrophine, et en particulier celle décrite dans le document Wang et al. (Gene Therapy, Vol. 15, No 22, pp 1489-1499, 2008).

Plus généralement est visée toute protéine présentant une activité thérapeutique dans une maladie neuromusculaire, par exemple dont la mutation cause une maladie au niveau d'un ou plusieurs tissus cibles, et ce dans le cas où sa production à partir d'un système d'expression présente une toxicité dans au moins un tissu, avantageusement un tissu non cible, en particulier au niveau du cœur, et de manière plus exhaustive dans au moins un tissu choisi dans le groupe suivant : le cœur, le foie, le cerveau, les poumons, le rein et les muscles lisses.

Selon l'invention, le système d'expression doit permettre l'expression à un niveau thérapeutiquement acceptable de la calpaine 3 dans les muscles squelettiques.

Par ailleurs, il doit permettre l'expression à un niveau toxiquement acceptable de la calpaine 3 au niveau du cœur.

Dans le cadre de la présente invention, le terme « expression de la protéine » peut s'entendre comme « production de la protéine ». Ainsi, le système d'expression doit permettre à la fois la transcription et la traduction de la protéine aux niveaux définis ci-dessus.

Les niveaux définis dans le cadre de l'invention, à savoir « thérapeutiquement acceptable » et « toxiquement acceptable » sont liés certes à la quantité de protéine mais également à son activité.

L'évaluation de la quantité de protéine produite dans un tissu donné peut être réalisée par détection immunologique à l'aide d'un anticorps dirigé contre ladite protéine, par exemple par Western blot ou ELISA. Alternativement, il peut être réalisé la quantification des ARN messagers correspondants, par exemple par PCR ou RT-PCR. Cette quantification peut être réalisée sur un échantillon du tissu, voire sur plusieurs échantillons. Ainsi et dans le cas où les tissus cibles sont les muscles squelettiques, celle-ci peut être réalisée au niveau d'un type musculaire ou de plusieurs types musculaires (par exemple quadriceps, diaphragme, tibial antérieur, triceps, ...).

Dans le cadre de l'invention, on entend par « niveau thérapeutiquement acceptable », le fait que la protéine produite à partir du système d'expression selon l'invention permet d'améliorer l'état pathologique du patient atteint, notamment en termes de durée de vie et de qualité de vie. Ainsi et en rapport avec une pathologie affectant les muscles squelettiques, il s'agit d'améliorer l'état musculaire du sujet atteint de la maladie, voire de restaurer un phénotype musculaire proche de celui d'un sujet sain. Comme mentionné ci-dessus, l'état musculaire, avantageusement défini par la force, la taille, l'histologie et la fonctionnalité des muscles, peut être évalué par l'une des méthodes suivantes : réalisation de biopsie, mesure de la force, de la tonicité, du volume, ou de la mobilité des muscles, examen clinique, imagerie médicale, biomarqueurs, ...

Ainsi, des critères permettant d'évaluer un bénéfice thérapeutique au niveau des muscles squelettiques et pouvant être évalués à différents temps après le traitement sont en particulier :
- l'augmentation de l'espérance de vie ;
- l'augmentation de la force musculaire
- l'amélioration de l'aspect histologique ; et/ou
- l'amélioration de la fonctionnalité du diaphragme.

Dans le cadre de l'invention, on entend par « niveau toxiquement acceptable », le fait que la protéine produite à partir du système d'expression selon l'invention n'entraîne pas d'altération majeure du tissu non cible, en particulier au niveau histologique, physiologique et/ou fonctionnel. En particulier, l'expression de la protéine ne doit pas être létale. Avantageusement, la quantité de protéine produite dans le tissu non cible ne doit pas dépasser le niveau endogène de ladite protéine dans ce tissu, notamment en comparaison avec un sujet sain. Comme déjà dit, la toxicité au niveau d'un tissu peut s'évaluer au niveau histologique, physiologique et fonctionnel. Dans le cas particulier du cœur et à titre d'illustration, la toxicité éventuelle d'une protéine peut être évaluée par l'étude de la morphologie et de la fonction cardiaque, par l'examen clinique, l'électrophysiologie, l'imagerie, des biomarqueurs, le suivi de l'espérance de vie ou par des analyses histologiques, notamment la détection de fibrose et/ou d'infiltrats cellulaires, par exemple par coloration au rouge sirius ou hematoxyline/éosine.

De manière avantageuse, le niveau d'efficacité et/ou de toxicité du système d'expression selon l'invention est évalué *in vivo* chez l'animal, encore plus avantageusement chez un animal présentant une copie défectueuse du gène codant la protéine et donc atteint de la pathologie associée.

Préférentiellement, le système d'expression est administré de manière systémique, par exemple par injection intraveineuse.

Il est décrit un système d'expression qui comprend au moins une séquence permettant :
- d'éviter l'expression ou de diminuer le niveau d'expression de la protéine dans les tissu non cibles, en particulier dans ceux où l'expression de la protéine est toxique ; et/ou
- de maintenir l'expression ou d'augmenter le niveau d'expression de la protéine dans le ou les tissus cibles.

Il est décrit un système d'expression comprenant au moins une séquence permettant :
- d'éviter l'expression ou de diminuer le niveau d'expression de la protéine dans les tissus autres que les muscles squelettiques et/ou le tissu nerveux périphérique, avantageusement dans ceux où l'expression de la protéine est toxique ; et/ou
- de maintenir l'expression ou d'augmenter le niveau d'expression de la protéine dans les muscles squelettiques et/ou le tissu nerveux périphérique.

Dans le cadre de l'invention, la terminologie « éviter l'expression » vise avantageusement le cas où, même en l'absence de ladite séquence, il n'y a pas d'expression, alors que la terminologie « diminuer le niveau d'expression » vise le cas où l'expression est diminuée (ou réduite) par l'apport de ladite séquence.

De manière similaire, la terminologie « maintenir l'expression » vise avantageusement le cas où, même en l'absence de ladite séquence, il y a expression à un niveau comparable, alors que la terminologie « augmenter le niveau d'expression » vise le cas où il y a une augmentation de l'expression par l'apport de ladite séquence.

Dans le cadre de la présente demande, il a été mis en évidence au moins trois moyens, éventuellement combinés, permettant d'atteindre l'objectif recherché :
- l'utilisation d'une séquence capable d'éviter l'expression ou de réduire le niveau d'expression de la protéine dans les tissus non cibles, sans réduire le niveau d'expression dans le ou les tissus cibles;
- l'utilisation d'une séquence promotrice capable d'assurer un niveau d'expression élevé dans le ou les tissus cibles et faible, voire nul, dans les tissus non cibles, en particulier dans ceux où l'expression de la protéine s'avère toxique ;
- l'utilisation d'un vecteur, avantageusement viral, présentant un tropisme adapté, en l'occurrence supérieur pour le ou les tissus cibles que pour les tissus non cibles, en particulier ceux où l'expression de la protéine s'avère toxique.

De manière caractéristique, un système d'expression selon l'invention comprend une séquence promotrice gouvernant la transcription de la séquence codant la calpaine 3, avantageusement placée en 5' de ce transgène et liée fonctionnellement à celle-ci. De manière avantageuse, celle-ci permet d'assurer un niveau d'expression thérapeutiquement acceptable de la protéine dans les muscles squelettiques.

Il peut aussi bien s'agir de promoteurs naturels ou synthétiques (artificiels), inductibles ou constitutifs. De la même manière, ils peuvent être de toute origine, notamment humaine, de la même origine que le transgène en présence ou d'une autre origine.

Selon un premier mode de réalisation, cette séquence promotrice correspond à un promoteur dit ubiquitaire ou non sélectif, c'est-à-dire présentant une spécificité tissulaire faible et assurant un niveau d'expression globalement similaire dans les différents tissus, aussi bien les tissus cibles que non cibles. A titre d'exemple peuvent être cités : le promoteur du cytomégalovirus (pCMV), le promoteur *de Mtm1.*

Selon un mode de réalisation particulier, il s'agit d'un promoteur adapté aux muscles squelettiques et/ou au tissu nerveux périphérique mais qui peut s'exprimer dans d'autres tissus, notamment dans d'autres muscles. A titre d'exemple peuvent être cités : le promoteur de la desmine, avantageusement de séquence SEQ ID NO : 11, le promoteur de l'alpha-actine squelettique, de la créatine kinase musculaire (MCK), le promoteur synthétique C5-12, le promoteur synapsine I (Syn) ou le promoteur CK6. En rapport avec les muscles squelettiques, il peut également être cité les promoteurs troponin, myogenic factor 5 (Myf5), myosin light chain 1/3 fast (MLC1/3f), myogenic differentiation 1 (Myod1), myogenin (Myog), paired box gene 7 (Pax7), MEF2. En rapport avec le tissu nerveux périphérique, il peut également être cité les promoteurs P0 et MBP (Myelin Basic Protein).

Selon un mode de réalisation avantageux, la séquence promotrice du système d'expression est choisie pour son activité promotrice différentielle entre les tissus cibles et non cibles, en l'occurrence supérieure dans les tissus cibles. Dans ce cas de figure, cette séquence permet donc d'augmenter l'expression de la protéine dans les tissus cibles, avantageusement les muscles squelettiques et/ou le tissu nerveux périphérique, tout en évitant l'expression dans les tissus non cibles, en particulier dans ceux où l'expression de la protéine est toxique.

A titre d'exemple et dans le cas où les tissus cibles sont les muscles squelettiques, le promoteur est avantageusement un promoteur dit muscle-spécifique. Selon une autre caractéristique avantageuse, ledit promoteur présente une activité promotrice faible voire nulle dans les tissus non cibles, notamment dans le cœur, permettant une expression à un niveau toxiquement acceptable de la protéine dans ces tissus.

Selon un mode de réalisation particulier, ladite séquence promotrice peut correspondre au promoteur du gène de la calpaine 3, avantageusement d'origine humaine, encore plus avantageusement de séquence SEQ ID NO : 12. Une autre séquence promotrice adaptée est celle du miARN 206 (miR206), avantageusement d'origine humaine, encore plus avantageusement de séquence SEQ ID NO : 13.

Ainsi et dans le cadre de la présente demande, il a été montré au moins pour la calpaine 3, qu'un système d'expression comprenant la séquence codant cette protéine, placée sous le contrôle du promoteur de la calpaine 3 ou du miARN 206, était capable d'assurer l'expression à un niveau thérapeutiquement acceptable de la protéine dans les muscles squelettiques, et à un niveau toxiquement acceptable de la protéine dans le cœur et le foie.

Selon un autre aspect, il est donc décrit un système d'expression comprenant une séquence codant une protéine, placée sous le contrôle d'un promoteur présentant la séquence SEQ ID NO : 12 ou SEQ ID NO : 13. Des séquences promotrices dérivées des séquences SEQ ID NO : 12 et SEQ ID NO : 13 ou correspondant à un fragment de celles-ci mais présentant une activité promotrice comparable, notamment en termes de spécificité tissulaire et éventuellement d'efficacité, sont également visées.

Dans le cas où cette séquence promotrice ne permet pas l'expression à un niveau toxiquement acceptable de la protéine dans les tissus non cibles, celle-ci est avantageusement associée à une séquence ayant pour fonction de réduire le niveau d'expression de la protéine dans les tissus non cibles, avantageusement dans les tissus non cibles où l'expression de la protéine s'avère toxique.

Ainsi et à titre d'exemple, aussi bien dans le cas de la myotubularine que de la calpaine 3, il a été montré que l'utilisation d'un promoteur de la desmine présentait une toxicité cardiaque. Au contraire et conformément à l'invention, l'utilisation d'un promoteur de la desmine, avantageusement de séquence SEQ ID NO : 11, associée à au moins une séquence cible du miARN-208a, avantageusement de séquence SEQ ID NO : 10, permet à la fois :
- un niveau d'expression thérapeutiquement acceptable de la protéine au niveau du tissu cible, avantageusement les muscles squelettiques ;
- un niveau d'expression toxiquement acceptable de la protéine au niveau des tissus non cibles, avantageusement le cœur, voire le foie.

Comme déjà dit, ladite séquence est capable d'éviter l'expression ou de réduire le niveau d'expression de la protéine dans les tissus non cibles, avantageusement dans le ou les tissus non cibles où l'expression de la protéine est toxique. Cette action peut s'exercer selon différents mécanismes, notamment :
- au niveau de la transcription de la séquence codant la protéine ;
- au niveau des transcrits résultant de la transcription de la séquence codant la protéine, par exemple via leur dégradation ;
- au niveau de la traduction des transcrits en protéine.

Une telle séquence est avantageusement une cible pour une petite molécule d'ARN choisie dans le groupe suivant :
- microARNs (« microRNAs) ;
- petits ARNs interférents endogènes (endogenous small interfering RNA ou siRNAs);
- petits fragments de l'ARN de transfert (tRNA);
- ARN des régions intergéniques ;
- ARN ribosomal (rRNA) ;
- Petits ARN nucléaires (snRNA) ;
- Petits ARN nucléolaires (snoRNA) ;
- ARN interagissant avec les protéines piwi (piRNA) ;

De manière avantageuse, cette séquence permet de maintenir l'expression, voire même d'augmenter le niveau d'expression de la protéine dans le ou les tissus cibles, avantageusement dans les muscles squelettiques.

Préférentiellement, une telle séquence est choisie pour son efficacité dans le tissu non cible dans lequel l'expression de la protéine s'avère toxique. L'efficacité de cette séquence pouvant être variable en fonction des tissus, il peut donc être nécessaire d'associer plusieurs de ces séquences, choisies pour leur efficacité dans l'ensemble des tissus non cibles visés où une toxicité est avérée.

Selon un mode de réalisation préféré, cette séquence est une séquence cible pour un microARN (miARN). De manière connue, une telle séquence judicieusement choisie permet de réprimer spécifiquement l'expression génique dans des tissus sélectionnés.

Ainsi et selon un mode de réalisation particulier, le système d'expression comprend une séquence cible pour un microARN (miARN) exprimé ou présent dans le ou les tissus non cibles dans lesquels l'expression de la protéine s'avère toxique, par exemple dans le cœur. De manière adaptée, la quantité de ce miARN présente dans le tissu cible, avantageusement dans les muscles squelettiques, est inférieure à celle présente dans le tissu non cible, voire même ce miARN n'est pas exprimé dans le tissu cible. Selon un mode de réalisation particulier, le miARN ciblé est spécifiquement exprimé dans le tissu non cible visé, par exemple le cœur.

De manière connue pour l'homme du métier, la présence ou le niveau d'expression, notamment dans un tissu donné, d'un miARN d'intérêt peut être évalué par PCR, avantageusement par RT-PCR, ou par Northern blot.

Les différents miRNA aujourd'hui identifiés, ainsi que leur séquence cible et leur spécificité tissulaire, sont connus de l'homme du métier et sont par exemple décrits dans le document WO 2007/000668.

Selon l'invention, le système d'expression comprend la séquence cible du miARN-208a (également notée miR208a, SEQ ID NO : 9). Avantageusement, cette séquence, identique chez l'homme, le chien et la souris, présente la séquence SEQ ID NO : 10 de 22 pb. Bien évidemment, toute séquence dérivée ou tronquée, reconnue par le miARN-208a peut être mis en œuvre dans le cadre de l'invention. Ainsi, il a été montré dans le cadre de la présente demande que l'utilisation de cette séquence cible, aussi bien en rapport avec la myotubularine que la calpaine 3, permettait de résoudre le problème de leur toxicité cardiaque voire hépatique dans le cas de la calpaine 3.

Comme déjà dit, une séquence cible pour un microARN peut être utilisée seule ou en combinaison avec d'autres séquences, avantageusement des séquences cibles pour un microARN, identiques ou différentes. Ces séquences peuvent être utilisées en tandem ou en orientation inverse.

Selon un mode de réalisation privilégié, notamment pour la séquence cible du miARN-208a, une (1) ou plusieurs, notamment deux (2) ou quatre (4) séquences, peuvent être mises en œuvre. Avantageusement, celles-ci sont utilisées en tandem, c'est-à-dire toutes dans la même orientation. Dans le cas où plusieurs séquences cibles sont mises en œuvre, celles-ci peuvent être séparées par un espaceur d'ADN de séquence aléatoire, de manière connue de l'homme du métier.

De manière avantageuse, dans le cas d'une séquence cible d'un miARN, notamment du miR208a, celle-ci est placée en 3' de la séquence codant la protéine, encore plus avantageusement insérée dans la région 3' UTR (« Untranslated Région ») du système d'expression, avantageusement de l'ADNc codant la protéine. Encore plus avantageusement et dans le cas où le système d'expression comprend un signal de polyadénylation en 3' de l'ADNc codant la protéine, cette séquence est insérée entre le codon stop du cadre ouvert de lecture et le signal de polyadénylation.

Dans le cadre de la présente demande, il a été mis en évidence qu'au moins une séquence cible du miARN-208a était adaptée pour obtenir un niveau toxiquement acceptable de protéine au moins dans le cœur, notamment concernant la myotubularine et la calpaine 3.

Selon un mode de réalisation particulier, il est décrit un système d'expression qui comprend :
- une séquence codant la myotubularine placée sous le contrôle d'un promoteur, avantageusement celui de la desmine, encore plus avantageusement celui de la desmine humaine (SEQ ID NO : 11);
- au moins une séquence cible d'un miARN exprimé dans le cœur, avantageusement du miARN-208a, avantageusement une seule séquence cible telle que la séquence SEQ ID NO : 10.

Selon l'invention, le système d'expression comprend :
- une séquence codant la calpaine 3 placée sous le contrôle d'un promoteur, avantageusement celui de la desmine, encore plus avantageusement celui de la desmine humaine (SEQ ID NO : 11), ou celui de la calpaine 3, encore plus avantageusement celui de la calpaine 3 humaine (SEQ ID NO : 12), ou celui du miRNA206, encore plus avantageusement celui du miRNA206 humain (SEQ ID NO : 13);
- au moins une séquence cible du miARN-208a, encore plus avantageusement deux séquences cibles en tandem.

Ainsi, les différents types de séquences détaillés ci-dessus peuvent être combinés dans un même système d'expression.

Selon la présente demande, un système d'expression ou cassette d'expression comprend les éléments nécessaires à l'expression du transgène présent. Outre les séquences telles que définies ci-dessus permettant d'assurer et de moduler l'expression du transgène, un tel système peut comprendre d'autres séquences telles que :
- un signal de polyadénylation, par exemple le polyA du SV40 ou de l'hémoglobine notamment humaine, avantageusement inséré en 3' de la séquence codante, voire en 3' de la séquence cible du miARN ;
- des séquences pour stabiliser les transcrits, telles que l'intron 1 de l'hémoglobine notamment humaine ;
- de séquences amplificatrices ou « enhancer » ;

Un système d'expression selon l'invention peut être introduit aussi bien dans une cellule, un tissu ou un organisme, notamment chez l'homme. De manière connue de l'homme du métier, l'introduction peut se faire *ex vivo* ou *in vivo,* par exemple par transfection ou transduction. Selon un autre aspect, il est décrit une cellule ou un tissu, avantageusement d'origine humaine, comprenant un système d'expression selon l'invention.

Le système d'expression selon l'invention, en l'espèce un acide nucléique isolé, peut être administré en l'état chez un sujet, à savoir sous la forme d'un ADN nu. Pour faciliter l'introduction de cet acide nucléique dans les cellules, celui-ci peut être associé à différents moyens chimiques tels que des systèmes de dispersion colloïdale (complexes macromoléculaires, nanocapsules, microsphères, billes) ou des systèmes basés sur les lipides (émulsions huile-dans-eau, micelles, liposomes).

Alternativement et selon un autre mode de réalisation privilégié, le système d'expression selon l'invention comprend un plasmide ou un vecteur. De manière avantageuse, un tel vecteur est un vecteur viral. Des vecteurs viraux couramment utilisés en thérapie génique chez les mammifères, notamment chez l'homme, sont connus de l'homme du métier. De tels vecteurs viraux sont avantageusement choisis dans la liste suivante : vecteur dérivé du virus de l'herpès, vecteur baculoviral, vecteur lentiviral, vecteur rétroviral, vecteur adénoviral et vecteur viral adéno-associé ou AAV (« adeno-associated virus »).

De manière préférée, il s'agit un vecteur viral adéno-associé ou AAV (« adeno-associated virus »), correspondant aussi bien à des sérotypes naturels (AAV1, AAV2, AAV3, AAV4, AAV5, AAV6, AAV7, AAV8 et AAV9), à des variants de ceux-ci ou à des sérotypes artificiels. De manière connue de l'homme du métier, des vecteurs AAV chimériques peuvent également être mis en œuvre.

Avantageusement, le système d'expression selon l'invention est inséré entre deux séquences ITR (« Inverted Terminal Repeat ») du vecteur AAV.

En rapport avec une administration systémique pour laquelle le système d'expression selon l'invention prend tout son sens, des vecteurs AAV de sérotype 8 ou 9 sont particulièrement privilégiés. Il peut par exemple s'agir de vecteurs AAV2/8 ou AAV2/9.

De manière connue de l'homme du métier, des particules virales recombinantes peuvent être obtenue, par exemple par tri-transfection de cellules 293 HEK ou par le système baculovirus. Les titres en vecteur sont classiquement exprimés en termes de génomes viraux par ml (vg/ml).

Selon un mode de réalisation privilégié, le système d'expression selon l'invention comprend un vecteur présentant un tropisme adapté, en l'occurrence supérieur pour le ou les tissus cibles que pour les tissus non cibles, en particulier ceux où l'expression de la protéine s'avère toxique. Il peut s'agir d'un vecteur AAV contenant une capside sélectionnée pour peu ou ne pas cibler/transduire, les tissus non cibles tels que le cœur, voire pour cibler/transduire spécifiquement les tissus cibles, en particulier les muscles squelettiques.

Comme il ressort de ce qui précède, les systèmes d'expression selon l'invention, notamment sous la forme de vecteurs AAV recombinants ou de particules virales recombinantes, ont des applications évidentes, notamment dans le domaine thérapeutique.

Ainsi et selon un autre aspect, l'invention concerne l'utilisation du système d'expression décrit comme médicament. En d'autres termes, une composition pharmaceutique comprenant un tel système d'expression est également visée. De manière adaptée, celle-ci peut en outre comprendre un véhicule pharmaceutiquement acceptable et inerte, avantageusement adapté à une administration systémique, par exemple intraveineuse. Divers excipients, stabilisateurs, et autres composés adaptés connus de l'homme du métier peuvent être ajoutés à une telle composition.

La présente invention a mis en évidence l'intérêt du système d'expression décrit dans le cas où son administration ne se fait pas de manière locale au niveau des tissus cibles, mais au contraire de manière générale au niveau du corps entier, aboutissant à sa délivrance au niveau de tissus non cibles.

Ainsi et de manière avantageuse, un système d'expression selon l'invention est administré par l'une des voies suivantes : administration entérale, parentérale, orale, intraveineuse, intra-artérielle et par inhalation.

De manière privilégiée, il s'agit d'une administration systémique, avantageusement une injection intraveineuse. A noter qu'une administration systémique peut être réalisée à proximité d'un site de traitement, par exemple à proximité d'un muscle squelettique.

Selon un mode de réalisation particulier, il ne s'agit pas d'une administration loco-régionale. Plus précisément, peut ainsi être exclue une injection intravasculaire notamment intraveineuse (réalisée sous pression, et en présence d'un garrot, à proximité du muscle ciblé), telle que décrite par exemple par Petrov et al. (Methods Mol Biol 2011 ; 709 : 277-86), ou intra-artérielle (cathéter dans une artère et clampage des veines à proximité ainsi que de l'artère, en amont, pour éviter la diffusion) telle que décrite par exemple par Gonin et al. (J Gene Med 2005 ; 7 : 782-791).

Lorsque la composition selon l'invention est à injecter, elle se présente préférentiellement sous forme liquide. La concentration en actif, en l'occurrence le système d'expression selon l'invention, la quantité à injecter et la fréquence des injections sont déterminées par l'homme du métier. Une administration unique peut s'avérer suffisante. Un effet thérapeutique est avantageusement recherché pour une durée d'au moins 1 mois, 3 mois, 6 mois, 1 an, 5 ans ou plus.

De tels médicaments sont notamment destinés à la thérapie génique, en particulier pour le traitement des maladies neuromusculaires et encore plus particulièrement des maladies affectant essentiellement les muscles squelettiques (myopathies). Plus généralement, l'invention permet notamment d'améliorer la fonction musculaire chez un sujet.

Les patients à traiter sont avantageusement des mammifères, en particulier des humains.

Une maladie particulièrement visée dans la demande est la myopathie centronucléaire, plus précisément la myopathie myotubulaire liée au chromosome X (XLMTM). Par ailleurs, d'autres myopathies centronucléaires et maladies neuromusculaires associées à la myotubularine, tels que certaines formes de la maladie de Charcot-Marie-Tooth, peuvent être traitées.

La dystrophie des ceintures de type 2A (LGMD2A) peut également être traitée par un système d'expression selon l'invention.

Plus généralement, une liste non exhaustive des pathologies visées est la suivante : dystrophie musculaire congénitale avec déficit en sélénoprotéine N, dystrophie musculaire congénitale avec déficit primaire en mérosine, dystrophie musculaire congénitale de type Ullrich, dystrophie musculaire de Duchenne (DMD) ou de Becker (BMD), myopathie congénitale à core centraux, myopathie congénitale à multi-minicores, myopathies centronucléaires autosomiques, myopathie avec dysproportion de types de fibres, myopathie à bâtonnets, syndromes myasténiques congénitaux, autres maladies neuromusculaires associées aux myotubularines, dystrophie des ceintures type 2B ou 2D, myopathie distale de type miyoshi, , dysferlinopathies, sarcoglycanopathies.

Selon un mode de réalisation particulier, la pathologie n'est pas la dystrophie musculaire de Duchenne (DMD) ou de Becker (BMD), voire la LGMD2D.

Il est donc attendu du médicament selon l'invention à la fois une amélioration de la pathologie, et ainsi de la qualité de vie et de la longévité du patient, tout en évitant les effets secondaires potentiels au niveau des autres tissus d'un tel traitement.

Comme il va être démontré à titre d'exemple, la présente demande a mis en évidence la possible toxicité cardiaque des traitements par thérapie génique des maladies musculaires et propose des solutions techniques permettant de surmonter ce problème.

### EXEMPLES DE REALISATION

L'invention et les avantages qui en découlent ressortiront mieux des exemples de réalisation suivants, à l'appui des figures annexées. Ceux-ci n'ont toutefois aucune portée limitative.

La présente invention est illustrée en rapport avec le gène de la myotubularine (*Mtm1*) et de la calpaine 3 *(CAPN3).* Toutefois, la stratégie décrite peut être appliquée à tout transgène codant une protéine d'intérêt au niveau des muscles squelettiques dont une toxicité notamment cardiaque est révélée.
Figure 1 (**hors invention)** : Schéma des constructions vectorielles :
   A/ cassette d'expression de *Mtm1* dépourvue de séquences cibles pour le miARN-208a;
   B/ cassette d'expression contenant 1, 2 ou 4 séquences cibles pour le miARN-208a (boîtes) en 3' du gène *Mtm1.*
Figure 2 (**hors invention)** : Section transversale du cœur d'une souris XLMTM traitée à l'aide d'un vecteur AAV-pDES-Mtm1. Les zones de fibrose sont révélées en rouge grâce à une coloration au rouge sirius.
Figure 3 :
   - en haut : distribution du vecteur dans les muscles squelettiques (muscle tibial antérieur = TA ; quadriceps = QUA ; triceps = TRI) et dans le cœur d'une souris sauvage (WT), 1 mois après l'administration intraveineuse des vecteurs (vg/génome diploïde).
   - en bas : niveau de protéine MTM1 dans les muscles squelettiques et le cœur d'une souris sauvage (WT), 1 mois après l'administration des vecteurs. Les valeurs indiquent le taux de multiplication par rapport aux niveaux endogènes. En tant que contrôles, des souris ont été injectées à l'aide soit de PBS soit du vecteur AAV8 vide (AAV-MCS).

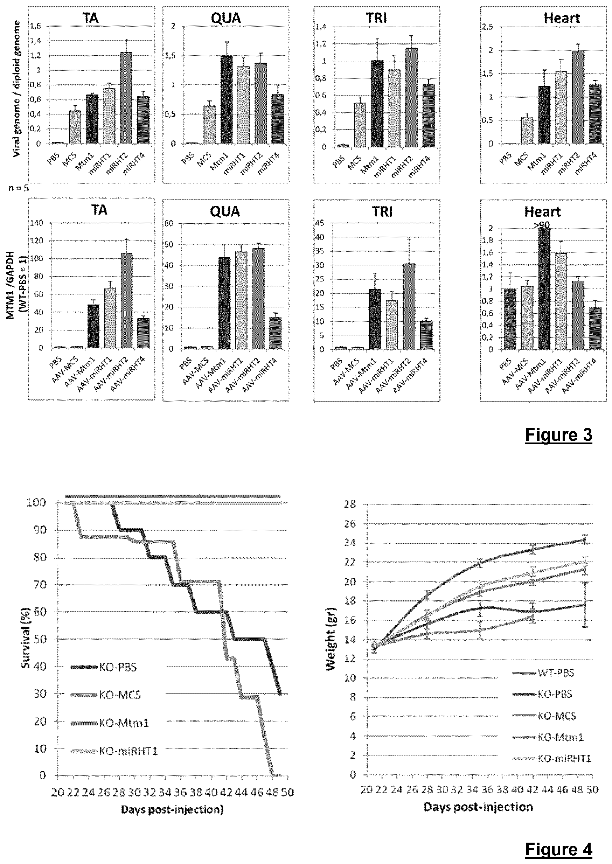

Figure 4 **(hors invention)** : Courbe de survie (à gauche) et courbe de masse corporelle (à droite) de souris KO (« Knock Out ») *Mtm1* injectée à l'aide soit de PBS, AAV8-Des-MCS, AAV8-Des-Mtm1 ou AAV8-Des-Mtm1-miRHT1. Des souris sauvages (WT) ont reçu du PBS comme contrôle.

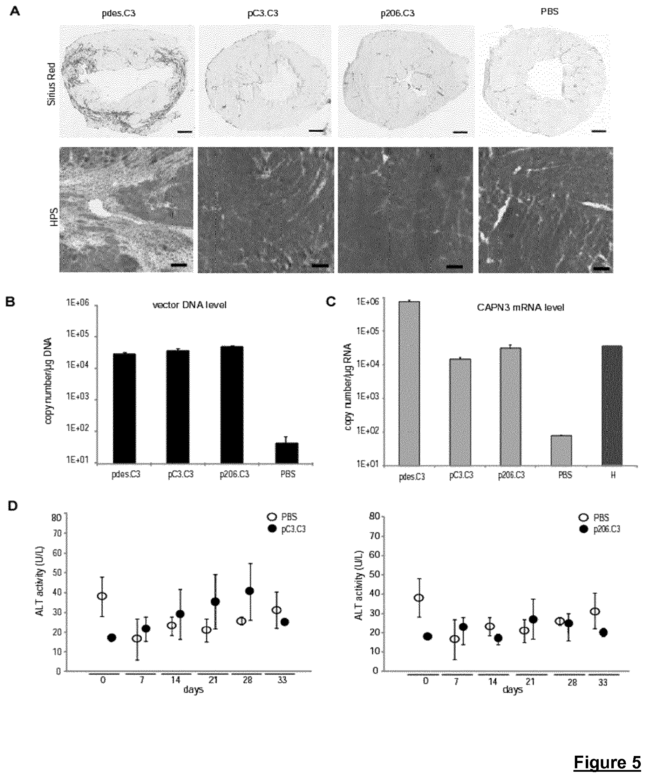

Figure 5 : Analyse des activités promotrices de *CAPN3* et miR-206 *in vivo :*
   A/ Analyse histologique du muscle cardiaque après injection de PBS ou des vecteurs AAV2/9-desm-CAPN3 (pdes.C3), AAV2/9-pC3-CAPN3 (pC3.C3), AAV2/9-pmiR206-CAPN3 (p206.C3) et coloration au rouge sirius (haut, échelle = 500µm) ou Hematoxyline Phloxine Saffron (HPS) (en bas, échelle = 100µm).
   B/ Evaluation du niveau d'ADN vectoriel par qPCR dans le cœur de souris sauvages WT après injection.
   C/ Evaluation du niveau d'ARNm *CAPN3* par qPCR dans le cœur de souris sauvages WT après injection. La ligne "H" correspond au niveau endogène d'ARNm CAPN3 dans le cœur de souris sauvages WT.
   D/ Analyse d'enzymes sériques. Les essais d'alanine aminotransférase (ALT) ont été réalisés sur des sérums de souris WT traitées avec pC3.C3, à gauche ou p206.C3, à droite. La moyenne et l'écart standard type (SEM) pour chaque condition sont indiqués par un cercle et une barre verticale, respectivement.

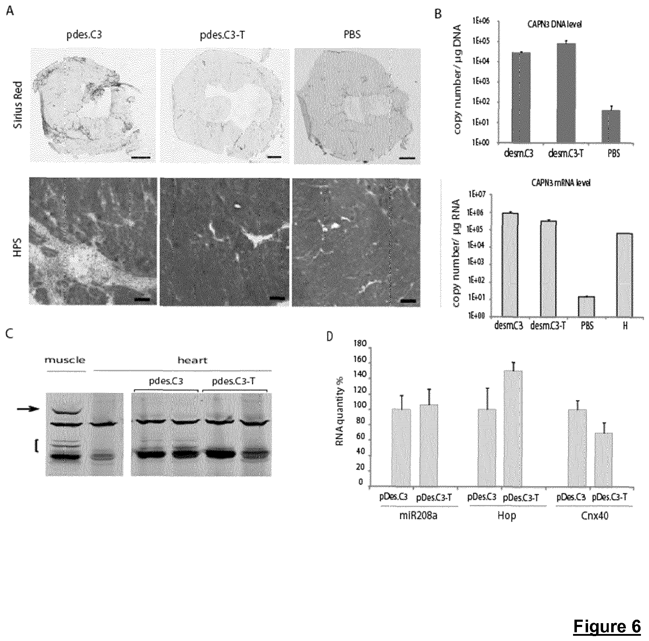

Figure 6 : Analyse de l'activité de miR-208aT *in vivo :*
   A/ Analyse histologique du muscle cardiaque, 35 jours après injection de PBS ou de doses identiques des vecteurs AAV2/9-desm-CAPN3 (pdes.C3) ou AAV2/9-desmin-CAPN3-miR208aT (pdes.C3-T) et coloration au rouge sirius (en haut, échelle = 500µm) ou HPS (en bas, échelle = 100µm).
   B/ Evaluation du niveau d'ADN vectoriel par qPCR dans le cœur de souris WT après injection (en haut) et du niveau d'ARNm du transgène *CAPN3* (en bas). La ligne "H" correspond au niveau endogène d'ARNm CAPN3 dans le cœur de souris sauvages WT.
   C/ Analyse de l'expression de la calpaine 3 par western blot dans le muscle squelettique et le cœur de souris WT injectées avec du PBS ou des vecteurs AAV2/9-desmin-CAPN3 (pdes.C3) ou AAV2/9-desm-CAPN3-miR208aT (pdes.C3-T). La protéine entière est indiquée par une flèche et ses produits de clivage (60, 58 and 55 kDa) par un crochet.
   D/ Quantification des niveaux d'ARNm de miR-208a (miR208a), HOP (Hop) et la connexine 40 (Cnx40) dans le cœur de souris WT injectées par AAV2/9-desmin-CAPN3 (pdes.C3) ou AAV2/9-desmin-CAPN3-miR208aT (pdes.C3-T). La quantité d'ARN dans la condition pdes.C3-T est donnée en pourcentage par rapport au niveau d'ARN dans la condition pdes.C3.

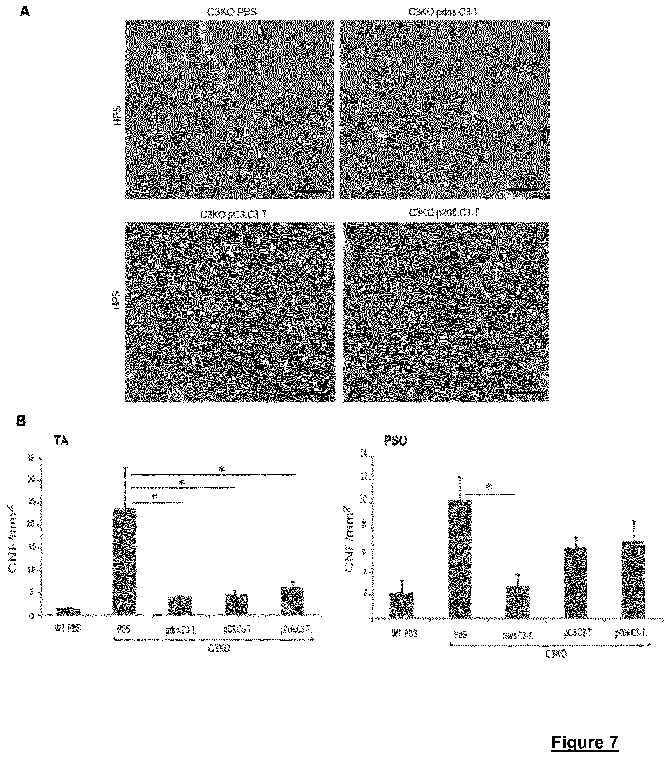

Figure 7 : Analyse histologique de l'efficacité du transfert de la calpaine 3 dans les muscles squelettiques de souris déficientes en calpaine 3 :
   A/ Des sections transversales des muscles TA de souris C3KO ont été colorées au HPS, 4 mois après injection soit avec du PBS ou des vecteurs (1.2 x 10¹³ vg/kg) AAV2/9-desmin-CAPN3-miR208aT (pdes.C3-T), AAV2/9-pC3-CAPN3-miR208aT (pC3.C3-T), AAV2/9-pmiR206-CAPN3-miR208aT (p206.C3-T). Echelle = 100 µm.
   B/ Nombre de fibres centronucléées (CNF/mm²) mesuré dans des sections colorées au HPS dans des muscles TA (à gauche) ou PSO (à droite) de souris C3KO injectées soit à l'aide de PBS ou des vecteurs (1.2 x 10¹³ vg/kg) AAV2/9-desmin-CAPN3-miR208aT (pdes.C3-T), AAV2/9-pC3-CAPN3-miR208aT (pC3.C3-T), AAV2/9-pmiR206-CAPN3-miR208aT (p206.C3-T). Une différence avec une valeur P < 0.05 est indiquée par un astérisque. TA: muscle tibial antérieur; PSO: muscle *Psoas.*

### I) MATERIEL ET METHODES

### 1) Génération des vecteurs AAV recombinants :

Le vecteur rAAV-Des-Mtm1 a été construit en clonant le cadre ouvert de lecture du gène murin *Mtm1* (SEQ ID NO: 14) en aval du promoteur humain de la desmine (SEQ ID NO: 11) dans un vecteur AAV de sérotype 2. Des séquences cibles (1, 2 ou 4 séquences, miRHT1, miRTH2 and miRHT4, respectivement) du miARN-208a de 22 pb (SEQ ID NO : 10), chacune séparée par des espaceurs d'ADN, ont été ajoutées dans la région 3'UTR de l'ADNc de *Mtm1.* Un vecteur vide (rAAV-Des-MCS) a également été généré comme contrôle. Des particules virales recombinantes de sérotype 8 (AAV8) ont été obtenues en utilisant un protocole de tri-transfection des cellules 293 HEK comme décrit précédemment (Buj Bello et al., 2008, Hum Mol Genet 17(14) : 2132-43). Les titres en vecteur sont exprimés en termes de génomes viraux par ml (vg/ml).

De manière similaire, le vecteur rAAV-desm-CAPN3 (ou AAV-desmin-CAPN3 ou AAV-pDes-CAPN3) a été construit en utilisant l'ADNc de la calpaine 3 humaine (SEQ ID NO: 8) sous le contrôle du promoteur humain de la desmine (SEQ ID NO : 11). Des vecteurs rAAV-pC3-CAPN3 et rAAV-pmiR206-CAPN3 ont été obtenus en remplaçant ce promoteur par la région promotrice de la calpaine 3 humaine (SEQ ID NO: 12) ou celle du miARN206 (SEQ ID NO: 13), respectivement. Des vecteurs AAV-desm-CAPN3-miR208aT, AAV-pC3-CAPN3-miR208aT et AAV-pmiR206-CAPN3-miR208aT ont été obtenus en ajoutant 2 séquences cibles pour le miARN208a (SEQ ID NO : 9) en tandem (miR208aT), en 3' du gène de la calpaine 3. Des particules virales recombinantes de sérotype 1 (AAV1), 8 (AAV8) et/ou 9 (AAV9) ont été produites.

### 2) Expériences in vivo:

Les souris ont été traitées selon les législations Française et Européenne concernant l'expérimentation animale. Dans cette étude, des souris sauvages WT C57B1/6 (Laboratoires Charles River) et une lignée de souris inactivée de manière constitutive pour la myotubularine (knockout) KO*-Mtm1,* également appelée BS53d4-129pas, ont été utilisées. Pour la calpaine 3, le modèle murin C3KO, décrit par Laure et al. (Febs J., 2010, 277: 4322-4337), a été utilisé.

Des vecteurs recombinants, aux doses indiquées, ont été injectés dans la veine de la queue des souris telles qu'indiqués (âgées de 3 semaines à 2 mois). Un volume équivalent de tampon salin (PBS) a été administré comme contrôle. Le statut clinique et le poids des animaux ont été suivis chaque semaine pour les animaux WT et trois fois par semaine pour les souris mutantes. Les souris ont été sacrifiées au temps indiqués.

### 3) Western blot:

Des muscles congelés à l'isopentane ont été coupés en sections transversales de 30 µm et lysés sur la glace dans un tampon contenant 150 mM NaCl, 10 mM Tris HCl (pH 7.4), 1 mM EGTA, 1 mM EDTA, 100 mM fluorure de sodium, 4 mM pyrophosphate de sodium, 2 mM orthovanadate de sodium, 1% Triton X100, et 0.5% IGEPAL supplémenté avec un cocktail complet d'inhibiteurs de protéases (Roche). Les extraits musculaires ont été incubés 1 h et centrifugés à 4° C à 12,000×g pendant 30 min. Les concentrations en protéines dans le surnageant ont été déterminées à l'aide du kit Bio-Rad "protein assay kit". Les protéines ont été soumises à une migration en SDS-PAGE et, après transfert sur une membrane de nitrocellulose, incubées avec des anticorps polyclonaux dirigés contre la myotubularine (p2348; Buj Bello et al., 2008, Hum Mol Genet 17(14) : 2132-43) et GAPDH (#MAB374, Millipore). Les bandes protéiques ont été visualisées par fluorescence infrarouge en utilisant le système "Odyssey Imaging System" (LICOR Biotechnology Inc.) et quantifiées à l'aide du programme "Odyssey Infrared Imaging System Software" (Application software, version 1.2, 2003).

Pour la détection de la calpaine 3, un protocole similaire a été mis en œuvre :
Les muscles ont été homogénéisés par FastPrep en utilisant le tampon de lyse suivant [20 mM Tris (pH 7,5), NaCl 150 mM, EGTA 2 mM, 0,1% de Triton X-100, 2 uM E64 (Sigma)] et des inhibiteurs de protéases (Complete mini protease inhibitor cocktail; Roche Applied Sciences ; 25 µl par mg de tissu). Les échantillons ont été traités avec 250U/100 µl de Benzonase (Calbiochem) pendant 30 min à 4 ° C pour digérer l'ADN. Les lysats musculaires ont été mélangés avec le tampon de charge [NuPage LDS (Invitrogen), TNT 3M (Sigma)], dénaturés pendant 10 minutes à 70 ° C et centrifugés brièvement. Les surnageants ont été séparés par gel de polyacrylamide NuPAGE Bis-Tris en gradient 4-12% (Invitrogen). Après transfert, les membranes ont été hybridés avec des anticorps contre la calpaine 3 (anticorps monoclonal de souris, Novocastra NCL-CALP-12A2, dilution 1/200), à 4 ° C pendant la nuit ou à température ambiante pendant 2-3 heures. Enfin, les membranes ont été incubées avec IRDye ® pour la révélation sur le scanner infrarouge Odyssey (LI-COR Biosciences, Lincoln, Nebraska, USA).

### 4) PCR :

### 4-1- Myotubularine (hors invention):

L'isolation de l'ADN à partir des muscles a été réalisée en utilisant le kit "Gentra Puregene Tissu Kit" (Qiagen), conformément aux instructions du fabricant. La concentration en ADN total a été déterminée en utilisant un spectrophotomètre Nanodrop ND-8000 (Nanodrop Technologies, France), et 80 ng d'ADN pour chaque échantillon a été utilisé comme matrice pour la PCR en temps réel. La PCR en temps réel Taqman a été réalisée sur chaque échantillon pour à la fois une partie du squelette commun au vecteur rAAV2/X pour déterminer les copies du génome viral, et du gène murin de la titine, pour standardiser le nombre de génomes murins présents dans chaque échantillon. Les amorces utilisées pour l'amplification des vecteurs rAAV ont été: 5'- CTCCATCACTAGGGGTTCCTTG -3' (forward ; SEQ ID NO: 15), 5'-GTAGATAAGTAGCATGGC -3' (reverse ; SEQ ID NO : 16). Les sondes MGB ont été doublement marquées (FAM-NFQ): 5'- TAGTTAATGATTAACCC -3' (probe; SEQ ID NO : 17). Des amorces et une sonde utilisées pour la titine ont été: 5'-AAAACGAGCAGTGACGTGAGC-3' (forward; SEQ ID NO: 18), 5'-TTCAGTCATGCTGCTAGCGC-3' (reverse; SEQ ID NO: 19), et 5'-TGCACGGAAGCGTCTCGTCTCAGTC-3' (probe; SEQ ID NO: 20) (Applied Biosystem). Les amplifications de la titine ont été réalisées en utilisant 80 ng d'ADN dilué dans un mélange "Absolute QPCR ROX Mix" (Thermo Fischer scientific), 0.1 µM de sondes Taqman et 0.2 µM d'amorces (forward and reverse), dans un volume final de 25 µl. Les conditions de cycles ont consisté en: une étape d'activation de l'ADN Polymérase Thermo-Start à 95°C pendant 15 min, suivie de 40 cycles de 2 étapes, 15 s de dénaturation à 95°C et 60 s d'hybridation et d'extension à 60°C. Les amplifications des rAAV ont été réalisées en utilisant 0.1 µM de sondes Taqman, 0.3 µM d'amorce reverse et 0.05µM d'amorce forward dans un volume final de 25 µl. Les conditions de cycles ont consisté en: une étape d'activation de l'ADN Polymérase Thermo-Start à 95°C pendant 15 min, suivie de 40 cycles de 2 étapes, 15 s de dénaturation à 95°C et 60 s d'hybridation et d'extension à 54°C. La PCR a été réalisée sur un thermocycleur 7900 HT (Applied Biosystem). Une gamme de dilution standard d'un plasmide contenant les séquences d'un squelette rAAV et de la titine a été utilisée sur chaque plaque de PCR en temps réel comme contrôle du nombre de copies. Tous les échantillons et les contrôles ont été dupliqués. Les données sont exprimées en nombre de copies du génome viral par génome diploïde.

### 4-2- Calpaine 3:

Les muscles ont été extraits à l'aide de la méthode Trizol (Invitrogen). Au cours de l'extraction, une fraction de l'échantillon a été conservée pour extraction d'ADN en vue de la quantification par PCR quantitative. L"ARN total a été extrait de l'extrait résiduel traité par le kit « ADN-free » (Ambion) pour éliminer l'ADN résiduel.
Pour la quantification de l'expression des microARN endogènes, 20 ng d'ARN total ont subis une transcription inverse en utilisant le kit "microARN TaqMan transcription inverse" (Applied Biosystems) et analysés par le test Taqman microARN ID511 pour miR-208a (Applied Biosystems). La normalisation des échantillons a été réalisée avec l'expression de snoRNA202 avec le test ID1232 (Applied Biosystems).
Pour l'amplification des ARNm de la calpaine 3 endogène ou transgénique, un µg d'ARN a été transcrit par transcription inverse en utilisant des hexamères aléatoires et oligodT et le kit Verso ADNc (Abgene) ou le kit « RevertAid H Minus First Strand cDNA Synthesis" (Fermentas). La PCR en temps réel a été réalisée par la méthode TaqMan ® en utilisant le système ABI PRISM 7700 (Applied Biosystems) et la solution « Absolute QPCR Rox Mix » (ABgene) à l'aide des paires d'amorces (.f et .r) et sonde Taqman (.p) suivantes : pour la quantification de la calpaine transgénique : CAPN3sfr.f (SEQ ID NO : 21) 5'_CGCCTCCAAGGCCCGT 3 '; CAPN3sfr.r (SEQ ID NO : 22) 5'_GGCGGAAGCGCTGGCT_3'; MGBTUCAPN3.p (SEQ ID NO : 23) 5'_CTACATCAACATGAGAGAGGT_3 ; pour la quantification de la calpaine humaine : CAPN3.f (SEQ ID NO : 24) 5'_CGCCTCCAAGGCCAGG 3 ', CAPN3.r (SEQ ID NO: 25) 5'_GGCGGAAGCGCTGGGA_3 et CAPN3.p (SEQ ID NO: 26) 5'_TACATCAACATGCGGGAGGT_3. Une dilution en série d'un ARN de contrôle a été utilisée dans chaque expérience et traitée avec les échantillons expérimentaux pour contourner la variabilité de l'efficacité de la préparation d'ADNc et de la PCR afin d'être en mesure de comparer les différentes expériences. Cet ARN a été préparé par une réaction de transcription *in vitro* à partir d'un plasmide portant une calpaine 3 ADNc muté et amplifiable par tous les couples d'amorces.
L'analyse de l'expression de la connexine 40 et HOP a été réalisée en utilisant les tests TaqMan ® Gene Expression (Applied Biosystens) suivants : pour Cnx40; Gja-5 [Mus musculus]: Mm00433619_s1 et hop: HOP homeobox [Mus musculus]: Mm00558630_m1. Les résultats de qRT-PCR sont exprimés en unité arbitraire rapportés à l'expression du gène murin ubiquitaire acide phosphoprotéine ribosomale (P0 GI: 15029771; MH181PO.F (SEQ ID NO: 27) 5'_CTCCAAGCAGATGCAGCAGA 3 '/ M267PO.R (SEQ ID NO : 28) 5'ACCATGATGCGCAAGGCTAT_3' / M225PO.p (SEQ ID NO : 29) 5 «_CCGTGGTGCTGATGGGCAAGAA_3 ').

### 5) Histologie :

Des cryosections transversales (8 µm d'épaisseur) des muscles cardiaque, hépatique ou squelettiques ont été colorées à l'aide d'hématoxyline d'éosine (HE), de rouge sirius ou d'Hématoxyline Phloxine Saffron (HFS) en utilisant des protocoles standards.

Les sections ont été montées avec le médium Eukitt (LABONORD). Les images numériques ont été capturées à l'aide d'une caméra CCD (Sony). Les analyses morphométriques des muscles squelettiques pour définir les nombre de fibres centronuclées (CNF/mm²) ont été effectuées à l'aide du logiciel Histolab (Microvision, Evry).

### 6) Mesure de l'activité ALT :

Des échantillons sanguins ont été prélevés sans coagulant. Après centrifugation (8000g, 10 min, 4°C), les sérums ont été analysés à l'aide du dispositif VITROS DT60 (Ortho Clinical Diagnostics, UK) à l'aide des cassettes « Vitros ALT DT slides » pour la détermination du taux d'alanine aminotransférase (ALT).

### II) RESULTATS

### A - MYOTUBULARINE (hors invention)

### 1) Toxicité cardiaque de la construction AAV-pDES-Mtm1 :

Il était connu du document Buj-Bello et al. (2008, Hum Mol Genet 17(14) : 2132-43) l'effet bénéfique d'une injection intramusculaire unique du gène de la myotubularine (Mtm1) placé sous le contrôle du promoteur CMV dans un vecteur AAV2/1.

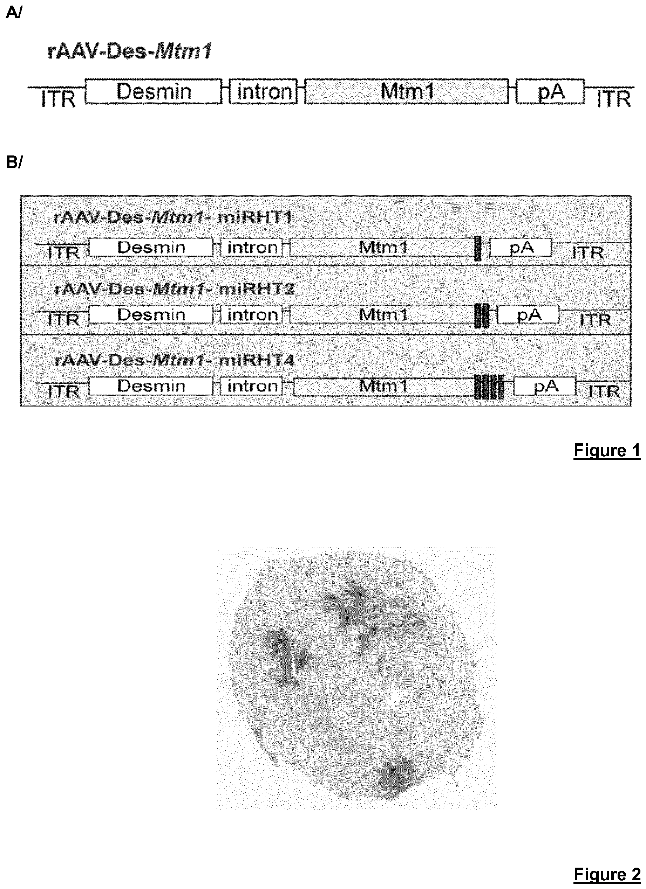

Une approche de thérapie génique par voie systémique chez des souris KO (« Knock Out ») *Mtm1* a été tentée et il a été montré que l'administration d'un vecteur AAV8 (rAAV-Des-Mtm1) exprimant la myotubularine sous le contrôle du promoteur humain de la desmine (Fig. 1A) chez une souris mutante aboutissait à une durée de vie prolongée d'au moins 6 mois, une amélioration robuste de la pathologie dans les muscles striés dans tout le corps y compris le diaphragme, et une activité motrice normalisée (résultats non montrés).

Toutefois, suite à une administration systémique du vecteur AAV8-DES-Mtm1 chez des souris KO *Mtml,* il a été observé que le niveau de protéine myotubularine était très élevé dans le cœur en comparaison avec les muscles squelettiques (résultats non montrés). De surcroit, il a été noté la présence d'infiltrats inflammatoires et de fibrose dans le cœur des souris XLMTM traitées à l'aide d'AAV à différents temps suivants l'injection virale (Figure 2).

### 2) Développements de systèmes d'expression sans toxicité cardiaque

Au vu de la difficulté pour prédire la biodistribution et l'expression du transgène à partir d'un vecteur AAV8 après administration systémique, notamment chez l'homme, de nouveaux vecteurs portant des séquences régulatrices augmentant la spécificité musculaire ont été développés, pour éviter les effets secondaires potentiels au niveau cardiaque.

Trois constructions virales (rAAV-Des-*Mtm1*-miRHT1 ; rAAV-Des-*Mtm1*-miRHT2 et rAAV-Des-*Mtm1*-miRHT4) ont été développées, telles que montrées à la Figure 1B, comprenant respectivement 1, 2 ou 4 séquences cibles pour le miARN-208a. Cette séquence présente la séquence SEQ ID NO : 10 et est constituée de 22 paires de bases.

De manière remarquable, cette séquence est conservée chez l'homme, le chien et la souris.

### 3) Production musculaire et cardiaque de MTM1 après injection chez une souris WT

Dans le but de sélectionner le vecteur d'expression le plus adapté pour MTM1, une dose unique de 3x10¹³ génomes viraux (vg)/kg de ces vecteurs a été administrée dans la veine de la queue de souris de type sauvage (WT pour « wild type ») âgées de 3 semaines. Un vecteur vide (AAV-Des-MCS) et du PBS (« Phosphate Buffered Saline ») ont été utilisés en tant que contrôles internes.

La distribution vectorielle et le niveau protéique en myotubularine dans le cœur et dans différents muscles squelettiques (Tibial antérieur = TA ; quadriceps = QUA ; triceps = TRI) ont été évalués 1 mois après injection. Des résultats par Western blot démontrent que ces vecteurs sont tous capables de diminuer le niveau de myotubularine produite à partir des vecteurs de manière spécifique dans le cœur. En outre, une seule séquence cible du miRNA208a est suffisante pour réduire l'expression dans ce tissu (Figure 3 et Tableau 1).

**Tableau 1 : Quantification semi-quantitative de la protéine MTM1 au niveau des muscles squelettiques et au niveau du cœur, 1 mois après la délivrance du vecteur chez une souris WT.**

| | | **PBS** | **Mtml** | **miRHT1** | **miRHT2** | **miRHT4** |
|---|---|---|---|---|---|---|
| Skeletal muscles | TA | 1 | 50 | 70 | 100 | 30 |
| | QUA | 1 | 45 | 45 | 50 | 15 |
| | TRI | 1 | 20 | 17 | 30 | 10 |
| H ea rt | | 1 | > 90 | 1.6 | 1.1 | 0.7 |

### 4) Validation de la construction vectorielle après injection chez une souris mutée Mtml

Sur la base des résultats précédents, la construction rAAV-Des-*Mtm1*-miRHT1 a été sélectionnée pour la suite des expériences. Des souris sauvages (WT pour « wild-type ») et mutées dans le gène *Mtm1* (KO pour « Knock Out ») ont reçu 3x10¹³ vg/kg de AAV-Des-*Mtm1*, rAAV-Des-*Mtm1*-miRHT1 et rAAV-Des-MCS, respectivement, ou du PBS à l'âge de 3 semaines, et ont été suivies au niveau clinique pendant 1 mois.

Toutes les souris mutantes qui ont reçu AAV8-Des-*Mtm1*-miRHT1 ont survécu jusqu'à la fin de l'étude, avec une courbe de croissance similaire à celles des souris KO traitées à l'aide de AAV8-Des-*Mtm1*, indiquant que l'inclusion de la séquence miRHT1 n'affecte pas l'efficacité thérapeutique du transgène (Fig. 4).

L'histologie du cœur des souris WT et KO a été analysée 1 mois après traitement, par coloration à l'hématoxyline-éosine et au rouge Sirius. Des régions de fibrose ont été observées dans le cœur de 7 souris KO sur 9 traitées avec AAV8-Des-*Mtm1*, mais pas chez les souris KO traitées avec AAV8-Des-*Mtm1*-miRHT1 (n=10). L'administration du vecteur AAV8-Des-*Mtm1* n'a pas entrainé de fibrose chez les animaux WT 1 mois après injection (n=8).

En conclusion, ces résultats indiquent que l'inclusion d'une seule séquence cible de miARN208a est suffisante pour réduire la toxicité cardiaque d'une construction AAV8-Des-*Mtm1*.
Des expériences similaires ont été réalisées en rapport avec la calpaine 3 (CAPN3) :

### B - CALPAINE 3

Il était connu du document Bartoli et al. (Molecular Therapy, 2006, Vol. 13, No 2, 250-259) l'effet bénéfique et la non toxicité de constructions de type AAV portant le gène de la calpaine 3 placé sous le contrôle de promoteurs dits muscle-spécifiques, après administration intramusculaire ou locale. Toutefois, les expériences réalisées ont révélé la toxicité de telles constructions après administration systémique :

### 1) Toxicité cardiaque des constructions AAV-desm-CAPN3 :

Le devenir des souris WT a été suivi, suite à l'injection intraveineuse de différentes constructions, et est présenté dans le tableau 2 ci-dessous :

**Tableau 2 : Conséquences des injections intraveineuses des différents AAV à différentes doses**

| **Sérotype** | **Dose (vg/kg)** | **Nombre de morts** | **Aspect histologique du cœur à 35 jours** |
|---|---|---|---|
| AAV9 | 4.0 x 10¹¹ | 0/3 | fibrose |
| " | 1.0 x 10¹² | 0/9 | fibrose |
| " | 1.6 x 10¹³ | 5/7 | fibrose |
| " | 4.3 x 10¹³ | 2/6 | fibrose |
| AAV8 | 7.0 x 10¹² | 2/4 | fibrose |
| AAV1 | 1.6 x 10¹³ | 0/3 | fibrose |

Pour tous les AAV testés, il est constaté une destruction du tissu cardiaque en cas d'administration systémique, excluant l'usage à visée thérapeutique de ces systèmes d'expression génique.

### 2) Réduction de la toxicité cardiaque des constructions AAV-desm-CAPN3 par remplacement du promoteur :

Deux vecteurs ont été construits en échangeant le promoteur de la desmine par celui de *CAPN3* (AAV2/9-pC3-CAPN3) ou miR-206 (AAV2/9-pmiR206-CAPN3). Après préparation virale des vecteurs, les conséquences *in vivo* des modifications introduites par injection intraveineuse (6 x 10¹² vg/kg) ont été analysées dans des souris C57BL/6 (WT) âgées de 2 mois.

35 jours après injection, aucune fibrose cardiaque n'a été observée chez les souris traitées avec les vecteurs AAV2/9-pC3-CAPN3 et AAV2/9-pmiR206-CAPN3, contrairement aux souris injectées avec AAV2/9-desm-CAPN3 (Fig. 5A), malgré un niveau similaire de transduction (Fig. 5B). Le niveau d'ARNm du transgène *CAPN3* dans le cœur de souris traitées avec AAV2/9-desm-CAPN3 était d'environ 15 fois plus élevé que le niveau endogène (Fig. 5C), alors qu'il est resté plus faible pour les souris traitées avec AAV2/9-pC3-CAPN3 et AAV2/9-pmiR206-CAPN3 (13% et 30%, respectivement, Fig. 5C), ce qui corrèle l'effet non délétère de ces deux derniers vecteurs.

Par ailleurs, il a été vérifié que ces deux promoteurs ne présentaient pas de toxicité hépatique en mesurant sur 5 semaines le niveau d'activité alanine aminotransférase (ALT) chez des souris WT ayant reçu une injection de 10¹³ vg/kg. Aucune augmentation de l'activité enzymatique n'a été observée chez les animaux injectés par rapport à ceux injectés avec du PBS (Fig. 5D).

En conclusion, les promoteurs *CAPN3* et miR-206 réduisent la toxicité cardiaque du transgène *CAPN3,* sans entraîner de toxicité hépatique.

### 3) Réduction de la toxicité cardiaque des constructions AAV-desm-CAPN3 par ajout de deux séquences cibles de miR208a :

Deux séquences cibles de miARN208a (SEQ ID NO : 10) ont été clonées en tandem dans une cassette miR208aT. Celle-ci a ensuite était introduite dans la région 3'UTR de la construction AAV2/9-desm-CAPN3, pour donner la construction AAV2/9-desm-CAPN3-miR208aT.

Après injection d'une dose de 6 x 10¹² vg/kg, aucune fibrose cardiaque n'a été observée chez les souris traitées, contrairement aux souris injectées avec AAV2/9-desm-CAPN3 (Fig. 6A), malgré un niveau de transduction similaire et un niveau d'ARNm 5 fois plus élevé par rapport au niveau endogène de la calpaine 3 dans le cœur (Fig. 6B). Au niveau protéique, la calpaine 3 n'est pas exprimée normalement au niveau du myocarde et n'est pas détectée (Fig. 6C). Dans les souris WT injectées avec AAV2/9-desm-CAPN3, la protéine entière n'est pas détectée mais les fragments résultant de son clivage (60, 58 et 55 kDa) le sont (Fig. 6C). Au contraire, ni protéine entière, ni fragments de clivage ne sont observés dans le cœur de souris WT injectées avec AAV2/9-desm-CAPN3-miR208aT (Fig. 6C), indiquant une régulation au niveau traductionnel (Fig. 6D).

En conclusion, ces résultats montrent que miR208aT est capable de réduire la toxicité cardiaque du transgène *CAPN3.*

### 4) Combinaison des deux stratégies :

De nouveaux vecteurs ont été construits en associant les promoteurs *CAPN3* et miR-206 et 2 copies de la séquence cible de miR-208a : AAV2/9-pC3-CAPN3-miR208aT et AAV2/9-pmiR206-CAPN3-miR208aT. Des souris C3KO (knockout pour la calpaine 3) ont reçu une injection de 1.2 x 10¹³ vg/kg de ces vecteurs.

Comme observé précédemment chez les souris sauvages, aucun des 3 vecteurs (AAV2/9-desm-CAPN3-miR208aT, AAV2/9-pC3-CAPN3-miR208aT et AAV2/9-pmiR206-CAPN3-miR208aT) ne s'est avéré toxique pour le cœur, 3 mois après l'injection (résultats non montrés).

En revanche, un examen histologique et morphologique des muscles squelettiques de souris C3KO âgées de 4 semaines et injectées à l'aide de ces vecteurs a révélé un effet positif de l'expression de la calpaine 3 sur les signes pathologiques du modèle murin. Les muscles du tibial antérieur (TA) injectés à l'aide de ces vecteurs ont montré des caractéristiques histologiques améliorés par rapport à ceux injectés avec du PBS (Fig. 7A). Une analyse morphométrique de sections de muscles TA colorés au HPS a révélé une diminution significative des fibres centronucléées (CNF) dans les muscles injectés à l'aide du vecteur (Fig. 9B à gauche). Des résultats similaires ont été obtenus avec les muscles PSO (muscle ilio-*psoas*) même si la diminution observée avec AAV2/9-pC3-CAPN3-miR208aT et AAV2/9-pmiR206-CAPN3-miR208aT n'était pas statistiquement significative.

En conclusion, ces résultats indiquent que l'expression de la calpaine 3 dans les muscles squelettiques transduits à l'aide de ces vecteurs recombinants peut corriger les signes pathologiques d'une souris déficiente en calpaine 3, sans présenter de toxicité cardiaque.

## Revendications

1. Système d'expression pour administration systémique comprenant une séquence codant la calpaine 3 placée sous le contrôle:
- d'une séquence promotrice permettant l'expression à un niveau thérapeutiquement acceptable de calpaine 3 dans les muscles squelettiques; et
- d'au moins une séquence cible du miR208a.

2. Système d'expression selon la revendication 1 ***caractérisé* en ce que** la calpaine 3 présente la séquence SEQ ID NO : 7.

3. Système d'expression selon l'une des revendications 1 à 2 ***caractérisé* en ce que** la séquence cible du miR208a présente la séquence SEQ ID NO : 10.

4. Système d'expression selon l'une des revendications 1 à 3 ***caractérisé* en ce qu'**il comprend 2 séquences cibles du miR208a, avantageusement en tandem.

5. Système d'expression selon l'une des revendications 1 à 4 ***caractérisé* en ce qu'**il comprend une séquence promotrice dite muscle-spécifique.

6. Système d'expression selon l'une des revendications 1 à 4 ***caractérisé* en ce que** la séquence promotrice est choisie dans le groupe suivant : la séquence promotrice du gène de la calpaine 3, avantageusement de séquence SEQ ID NO : 12, la séquence promotrice de miR206, avantageusement de séquence SEQ ID NO : 13, et la séquence promotrice du gène de la desmine, avantageusement de séquence SEQ ID NO : 11.

7. Système d'expression selon l'une des revendications précédentes ***caractérisé* en ce qu'**il comprend un vecteur viral, avantageusement un vecteur viral adéno-associé (AAV).

8. Système d'expression selon la revendication 7 ***caractérisé* en ce qu'**il comprend un vecteur AAV de sérotype AAV8 ou AAV9.

9. Composition pharmaceutique comprenant un système d'expression selon l'une des revendications 1 à 8.

10. Système d'expression selon l'une des revendications 1 à 8 pour son utilisation comme médicament.

11. Système d'expression selon l'une des revendications 1 à 8 pour son utilisation en thérapie génique.

12. Système d'expression selon l'une des revendications 1 à 8 pour son utilisation dans le traitement d'une maladie neuromusculaire, avantageusement d'une myopathie, encore plus avantageusement choisie dans le groupe suivant : LGMD2A, myopathie congénitale à multi-minicores et myopathies centronucléaires autosomiques.

13. Système d'expression pour son utilisation selon la revendication 11 ou 12 ***caractérisé* en ce que** le système d'expression est administré par voie systémique, avantageusement par injection intraveineuse.

## Patentansprüche

1. Expressionssystem zur systemischen Verabreichung, umfassend eine Calpain 3 codierende Sequenz, die unter der Kontrolle:
- einer Promotorsequenz, welche die Expression von Calpain 3 in Skelettmuskulatur auf therapeutisch akzeptablem Niveau ermöglicht; und
- mindestens einer Zielsequenz von miR208a
steht.

2. Expressionssystem gemäß Anspruch 1, **dadurch gekennzeichnet, dass** das Calpain 3 die Sequenz SEQ ID NO: 7 aufweist.

3. Expressionssystem gemäß einem der Ansprüche 1 bis 2, **dadurch gekennzeichnet, dass** die Zielsequenz von miR208a die Sequenz SEQ ID NO: 10 aufweist.

4. Expressionssystem gemäß einem der Ansprüche 1 bis 3, **dadurch gekennzeichnet, dass** es 2 Zielsequenzen von miR208a vorteilhafterweise im Tandem umfasst.

5. Expressionssystem gemäß einem der Ansprüche 1 bis 4, **dadurch gekennzeichnet, dass** es eine sogenannte muskelspezifische Promotorsequenz umfasst.

6. Expressionssystem gemäß einem der Ansprüche 1 bis 4, **dadurch gekennzeichnet, dass** die Promotorsequenz aus der folgenden Gruppe ausgewählt ist: die Promotorsequenz des Calpain-3-Gens, vorteilhafterweise von der Sequenz SEQ ID NO: 12, die Promotorsequenz von miR206, vorteilhafterweise von der Sequenz SEQ ID NO: 13, und die Promotorsequenz des Desmin-Gens, vorteilhafterweise von der Sequenz SEQ ID NO: 11.

7. Expressionssystem gemäß einem der vorhergehenden Ansprüche, **dadurch gekennzeichnet, dass** es einen viralen Vektor, vorteilhafterweise einen Adeno-assoziierten viralen Vektor (AAV), umfasst.

8. Expressionssystem gemäß Anspruch 7, **dadurch gekennzeichnet, dass** es einen AAV-Vektor vom Serotyp AAV8 oder AAV9 umfasst.

9. Pharmazeutische Zusammensetzung, umfassend ein Expressionssystem gemäß einem der Ansprüche 1 bis 8.

10. Expressionssystem gemäß einem der Ansprüche 1 bis 8 zur Verwendung als Arzneimittel.

11. Expressionssystem gemäß einem der Ansprüche 1 bis 8 zur Verwendung in der Gentherapie.

12. Expressionssystem gemäß einem der Ansprüche 1 bis 8 zur Verwendung bei der Behandlung einer neuromuskulären Erkrankung, vorteilhafterweise einer Myopathie, noch vorteilhafter ausgewählt aus der folgenden Gruppe: LGMD2A, kongenitale Multiminicore-Myopathie und autosomale zentronukleäre Myopathien.

13. Expressionssystem zur Verwendung gemäß Anspruch 11 oder 12, **dadurch gekennzeichnet, dass** das Expressionssystem auf systemischem Wege, vorteilhafterweise durch intravenöse Injektion, verabreicht wird.

## Claims

1. An expression system for systemic administration comprising a sequence encoding calpain 3 placed under the control of:
- a promoter sequence allowing the expression at a therapeutically acceptable level of calpain 3 in skeletal muscles; and
- at least one target sequence of miR208a.

2. The expression system according to claim 1, ***characterized* in that** the calpain 3 has the sequence SEQ ID NO: 7.

3. The expression system according to claim 1 or 2, ***characterized* in that** the target sequence of miR208a has the sequence SEQ ID NO: 10.

4. The expression system according to any of claims 1 to 3, ***characterized* in that** it comprises 2 target sequences of miR208a, advantageously in tandem.

5. The expression system according to any of claims 1 to 4, ***characterized* in that** it comprises a so-called muscle-specific promoter sequence.

6. The expression system according to any of claims 1 to 4, ***characterized* in that** the promoter sequence is chosen from the following group: the promoter sequence of the calpain 3 gene, advantageously of sequence SEQ ID NO: 12, the promoter sequence of miR206, advantageously of sequence SEQ ID NO: 13, and the promoter sequence of the desmin gene, advantageously of sequence SEQ ID NO: 11.

7. The expression system according to any of the preceding claims, ***characterized* in that** it comprises a viral vector, advantageously an adeno-associated viral vector (AAV).

8. The expression system according to claim 7, ***characterized* in that** it comprises an AAV vector of serotype AAV8 or AAV9.

9. A pharmaceutical composition comprising an expression system according to any of claims 1 to 8.

10. An expression system according to any of claims 1 to 8 for use as a medicament.

11. An expression system according to any of claims 1 to 8 for use in gene therapy.

12. An expression system according to any of claims 1 to 8 for use in the treatment of a neuromuscular disease, advantageously of a myopathy, even more advantageously chosen from the following group: LGMD2A, congenital myopathy with multi-minicores and autosomal centronuclear myopathies.

13. An expression system for its use according to claim 11 or 12, ***characterized* in that** the expression system is administered systemically, preferably by intravenous injection.


### SEQUENCE LISTING

<110> GENETHON
   CENTRE NATIONAL DE LA RECHERCHE SCIENTIFIQUE
<120> SYSTEME D'EXPRESSION POUR UNE THERAPIE GENIQUE SELECTIVE
<130> G143-B-33520 EP DIV
<140> PCT/FR/2014/050866
   <141> 2014-04-10
<150> FR1353306
   <151> 2013-04-11
<160> 29
<170> BiSSAP 1.2
<210> 1
   <211> 603
   <212> PRT
   <213> Homo sapiens
<220>
   <223> N° Accession NCBI: NP_000243.1
<400> 1
<210> 2
   <211> 603
   <212> PRT
   <213> Mus musculus
<220>
   <223> N° Accession NCBI: AAC77821.1
<400> 2
   Met Ala Ser Ala Ser Ala Ser Lys Tyr Asn Ser His Ser Leu Glu Asn
   1               5                   10                  15
   Glu Ser Ile Lys Lys Val Ser Gln Asp Gly Val Ser Gln Asp Val Ser
               20                  25                  30
   Glu Thr Val Pro Arg Leu Pro Gly Glu Leu Leu Ile Thr Glu Lys Glu
           35                  40                  45
   Val Ile Tyr Ile Cys Pro Phe Asn Gly Pro Ile Lys Gly Arg Val Tyr
       50                  55                  60
   Ile Thr Asn Tyr Arg Leu Tyr Leu Arg Ser Leu Glu Thr Asp Ser Ala
   65                  70                  75                  80
   Leu Ile Leu Asp Val Pro Leu Gly Val Ile Ser Arg Ile Glu Tyr Met
                   85                  90                  95
   Gly Gly Ala Thr Ser Arg Gly Glu Asn Ser Tyr Gly Leu Asp Ile Thr
               100                 105                 110
   Cys Lys Asp Leu Arg Asn Leu Arg Phe Ala Leu Lys Gln Glu Gly His
           115                 120                 125
   Ser Arg Arg Asp Met Phe Glu Ile Leu Val Lys His Ala Phe Pro Leu
       130                 135                 140
   Ala His Asn Leu Pro Leu Phe Ala Phe Val Asn Glu Glu Lys Phe Asn
   145                 150                 155                 160
   Val Asp Gly Trp Thr Val Tyr Asn Pro Val Glu Glu Tyr Arg Arg Gln
                   165                 170                 175
   Gly Leu Pro Asn His His Trp Arg Ile Ser Phe Ile Asn Lys Cys Tyr
               180                 185                 190
   Glu Leu Cys Glu Thr Tyr Pro Ala Leu Leu Val Val Pro Tyr Arg Thr
           195                 200                 205
   Ser Asp Asp Asp Leu Arg Arg Ile Ala Thr Phe Arg Ser Arg Asn Arg
       210                 215                 220
   Leu Pro Val Leu Ser Trp Ile His Pro Glu Asn Lys Met Val Ile Met
   225                 230                 235                 240
   Arg Cys Ser Gln Pro Leu Val Gly Met Ser Gly Lys Arg Asn Lys Asp
                   245                 250                 255
   Asp Glu Lys Tyr Leu Asp Val Ile Arg Glu Thr Asn Lys Gln Thr Ser
               260                 265                 270
   Lys Leu Met Ile Tyr Asp Ala Arg Pro Ser Val Asn Ala Val Ala Asn
           275                 280                 285
   Lys Ala Thr Gly Gly Gly Tyr Glu Ser Asp Asp Ala Tyr Gln Asn Ser
       290                 295                 300
   Glu Leu Ser Phe Leu Asp Ile His Asn Ile His Val Met Arg Glu Ser
   305                 310                 315                 320
   Leu Lys Lys Val Lys Asp Ile Val Tyr Pro Asn Ile Glu Glu Ser His
                   325                 330                 335
   Trp Leu Ser Ser Leu Glu Ser Thr His Trp Leu Glu His Ile Lys Leu
               340                 345                 350
   Val Leu Thr Gly Ala Ile Gln Val Ala Asp Gln Val Ser Ser Gly Lys
           355                 360                 365
   Ser Ser Val Leu Val His Cys Ser Asp Gly Trp Asp Arg Thr Ala Gln
       370                 375                 380
   Leu Thr Ser Leu Ala Met Leu Met Leu Asp Ser Phe Tyr Arg Thr Ile
   385                 390                 395                 400
   Glu Gly Phe Glu Ile Leu Val Gln Lys Glu Trp Ile Ser Phe Gly His
                   405                 410                 415
   Lys Phe Ala Ser Arg Ile Gly His Gly Asp Lys Asn His Ala Asp Ala
               420                 425                 430
   Asp Arg Ser Pro Ile Phe Leu Gln Phe Ile Asp Cys Val Trp Gln Met
           435                 440                 445
   Ser Lys Gln Phe Pro Thr Ala Phe Glu Phe Asn Glu Gly Phe Leu Ile
       450                 455                 460
   Thr Val Leu Asp His Leu Tyr Ser Cys Arg Phe Gly Thr Phe Leu Phe
   465                 470                 475                 480
   Asn Cys Asp Ser Ala Arg Glu Arg Gln Lys Leu Thr Glu Arg Thr Val
                   485                 490                 495
   Ser Leu Trp Ser Leu Ile Asn Ser Asn Lys Asp Lys Phe Lys Asn Pro
               500                 505                 510
   Phe Tyr Thr Lys Glu Ile Asn Arg Val Leu Tyr Pro Val Ala Ser Met
           515                 520                 525
   Arg His Leu Glu Leu Trp Val Asn Tyr Tyr Ile Arg Trp Asn Pro Arg
       530                 535                 540
   Val Lys Gln Gln Gln Pro Asn Pro Val Glu Gln Arg Tyr Met Glu Leu
   545                 550                 555                 560
   Leu Ala Leu Arg Asp Asp Tyr Ile Lys Arg Leu Glu Glu Leu Gln Leu
                   565                 570                 575
   Ala Asn Ser Ala Lys Leu Ala Asp Ala Pro Ala Ser Thr Ser Ser Ser
               580                 585                 590
   Ser Gln Met Val Pro His Val Gln Thr His Phe
   595 600
<210> 3
   <211> 603
   <212> PRT
   <213> Canis lupus
<220>
   <223> N° Accession NCBI : XP_855209.1
<400> 3
   Met Ala Ser Ala Pro Thr Ser Lys Tyr Asn Ser His Ser Leu Glu Asn
   1               5                   10                  15
   Glu Ser Ile Lys Arg Thr Ser Arg Asp Gly Val Asn Trp Asp Leu Ser
               20                  25                  30
   Glu Ala Val Pro Arg Leu Pro Gly Glu Thr Arg Ile Thr Asp Lys Glu
           35                  40                  45
   Val Ile Tyr Ile Cys Pro Phe Asn Gly Pro Ile Lys Gly Arg Val Tyr
       50                  55                  60
   Ile Thr Asn Tyr Arg Leu Tyr Leu Arg Ser Leu Glu Thr Asp Ser Ala
   65                  70                  75                  80
   Leu Ile Leu Asp Val Pro Leu Gly Val Ile Ser Arg Ile Glu Lys Met
                   85                  90                  95
   Gly Gly Ala Thr Ser Arg Gly Glu Asn Ser Tyr Gly Leu Asp Ile Thr
               100                 105                 110
   Cys Lys Asp Met Arg Asn Leu Arg Phe Ala Leu Lys Gln Glu Gly His
           115                 120                 125
   Ser Arg Arg Asp Met Phe Glu Ile Leu Thr Arg Tyr Ala Phe Pro Leu
       130                 135                 140
   Ala His Ser Leu Pro Ile Phe Ala Phe Leu Asn Glu Glu Lys Phe Asn
   145                 150                 155                 160
   Val Asp Gly Trp Thr Val Tyr Asn Pro Val Glu Glu Tyr Arg Arg Gln
                   165                 170                 175
   Gly Leu Pro Asn His His Trp Arg Ile Thr Phe Ile Asn Lys Cys Tyr
               180                 185                 190
   Glu Leu Cys Asp Thr Tyr Pro Ala Leu Leu Val Val Pro Tyr Arg Ala
           195                 200                 205
   Ser Asp Asp Asp Leu Arg Arg Val Ala Thr Phe Arg Ser Arg Asn Arg
       210                 215                 220
   Ile Pro Val Leu Ser Trp Ile His Pro Glu Asn Lys Thr Val Ile Val
   225                 230                 235                 240
   Arg Cys Ser Gln Pro Leu Val Gly Met Ser Gly Lys Arg Asn Lys Asp
                   245                 250                 255
   Asp Glu Lys Tyr Leu Asp Val Ile Arg Glu Thr Asn Arg Gln Ile Ser
               260                 265                 270
   Lys Leu Thr Ile Tyr Asp Ala Arg Pro Asn Val Asn Ala Val Ala Asn
           275                 280                 285
   Lys Ala Thr Gly Gly Gly Tyr Glu Ser Asp Asp Ala Tyr His Asn Ala
       290                 295                 300
   Glu Leu Phe Phe Leu Asp Ile His Asn Ile His Val Met Arg Glu Ser
   305                 310                 315                 320
   Leu Lys Lys Val Lys Asp Ile Val Tyr Pro Asn Val Glu Glu Ser His
                   325                 330                 335
   Trp Leu Ser Ser Leu Glu Ser Thr His Trp Leu Glu His Ile Lys Leu
               340                 345                 350
   Val Leu Thr Gly Ala Ile Gln Val Ala Asp Arg Val Ser Ser Gly Lys
           355                 360                 365
   Ser Ser Val Leu Val His Cys Ser Asp Gly Trp Asp Arg Thr Ala Gln
       370                 375                 380
   Leu Thr Ser Leu Ala Met Leu Met Leu Asp Ser Phe Tyr Arg Ser Ile
   385                 390                 395                 400
   Glu Gly Phe Glu Ile Leu Val Gln Lys Glu Trp Ile Ser Phe Gly His
                   405                 410                 415
   Lys Phe Ala Ser Arg Ile Gly His Gly Asp Lys Asn His Ala Asp Ala
               420                 425                 430
   Asp Arg Ser Pro Ile Phe Leu Gln Phe Ile Asp Cys Val Trp Gln Met
           435                 440                 445
   Ser Lys Gln Phe Pro Thr Ala Phe Glu Phe Asn Glu Arg Phe Leu Ile
       450                 455                 460
   Thr Ile Leu Asp His Leu Tyr Ser Cys Arg Phe Gly Thr Phe Leu Tyr
   465                 470                 475                 480
   Asn Cys Glu Ser Ala Arg Glu Lys Gln Lys Val Thr Glu Arg Thr Val
                   485                 490                 495
   Ser Leu Trp Ser Leu Ile Asn Ser Asn Lys Asp Lys Phe Lys Asn Pro
               500                 505                 510
   Phe Tyr Thr Lys Glu Ile Asn Arg Val Leu Tyr Pro Val Ala Ser Met
           515                 520                 525
   Arg His Leu Glu Leu Trp Val Asn Tyr Tyr Ile Arg Trp Asn Pro Arg
       530                 535                 540
   Ile Lys Gln Gln Gln Pro Asn Pro Val Glu Gln Arg Tyr Val Glu Leu
   545                 550                 555                 560
   Leu Ala Leu Arg Asp Glu Tyr Ile Gln Arg Leu Glu Glu Leu Gln Leu
                   565                 570                 575
   Ala Ser Ser Ala Lys Leu Pro Asp Pro Ser Thr Ser Pro Ala Gly Pro
               580                 585                 590
   Ser Gln Met Met Pro His Val Arg Thr His Phe
           595                 600
<210> 4
   <211> 3452
   <212> DNA
   <213> Homo sapiens
<220>
   <221> source
   <222> 1..3452
   <223> /mol_type="unassigned DNA" /note="N° Accession NCBI : NM_000252.2" /organism="Homo sapiens"
<400> 4
   agagggggcg gagcagggcc cggcagccga gcagcctggc aacggcggtg gcgcccggag      60
   cccgagagtt tccaggatgg cttctgcatc aacttctaaa tataattcac actccttgga     120
   gaatgagtct attaagagga cgtctcgaga tggagtcaat cgagatctca ctgaggctgt     180
   tcctcgactt ccaggagaaa cactaatcac tgacaaagaa gttatttaca tatgtccttt     240
   caatggcccc attaagggaa gagtttacat cacaaattat cgtctttatt taagaagttt     300
   ggaaacggat tcttctctaa tacttgatgt tcctctgggt gtgatctcga gaattgaaaa     360
   aatgggaggc gcgacaagta gaggagaaaa ttcctatggt ctagatatta cttgtaaaga     420
   catgagaaac ctgaggttcg ctttgaaaca ggaaggccac agcagaagag atatgtttga     480
   gatcctcacg agatacgcgt ttcccctggc tcacagtctg ccattatttg catttttaaa     540
   tgaagaaaag tttaacgtgg atggatggac agtttacaat ccagtggaag aatacaggag     600
   gcagggcttg cccaatcacc attggagaat aacttttatt aataagtgct atgagctctg     660
   tgacacttac cctgctcttt tggtggttcc gtatcgtgcc tcagatgatg acctccggag     720
   agttgcaact tttaggtccc gaaatcgaat tccagtgctg tcatggattc atccagaaaa     780
   taagacggtc attgtgcgtt gcagtcagcc tcttgtcggt atgagtggga aacgaaataa      840
   agatgatgag aaatatctcg atgttatcag ggagactaat aaacaaattt ctaaactcac      900
   catttatgat gcaagaccca gcgtaaatgc agtggccaac aaggcaacag gaggaggata      960
   tgaaagtgat gatgcatatc ataacgccga acttttcttc ttagacattc ataatattca     1020
   tgttatgcgg gaatctttaa aaaaagtgaa ggacattgtt tatcctaatg tagaagaatc     1080
   tcattggttg tccagtttgg agtctactca ttggttagaa catatcaagc tcgttttgac     1140
   aggagccatt caagtagcag acaaagtttc ttcagggaag agttcagtgc ttgtgcattg     1200
   cagtgacgga tgggacagga ctgctcagct gacatccttg gccatgctga tgttggatag     1260
   cttctatagg agcattgaag ggttcgaaat actggtacaa aaagaatgga taagttttgg     1320
   acataaattt gcatctcgaa taggtcatgg tgataaaaac cacaccgatg ctgaccgttc     1380
   tcctattttt ctccagttta ttgattgtgt gtggcaaatg tcaaaacagt tccctacagc     1440
   ttttgaattc aatgaacaat ttttgattat aattttggat catctgtata gttgccgatt     1500
   tggtactttc ttattcaact gtgaatctgc tcgagaaaga cagaaggtta cagaaaggac     1560
   tgtttcttta tggtcactga taaacagtaa taaagaaaaa ttcaaaaacc ccttctatac     1620
   taaagaaatc aatcgagttt tatatccagt tgccagtatg cgtcacttgg aactctgggt     1680
   gaattactac attagatgga accccaggat caagcaacaa cagccgaatc cagtggagca     1740
   gcgttacatg gagctcttag ccttacgcga cgaatacata aagcggcttg aggaactgca     1800
   gctcgccaac tctgccaagc tttctgatcc cccaacttca ccttccagtc cttcgcaaat     1860
   gatgccccat gtgcaaactc acttctgagg ggggaccctg gcaccgcatt agagctcgaa     1920
   ataaaggcga tagctgactt tcatttgggg catttgtaaa aagtagatta aaatatttgc     1980
   ctccatgtag aacttgaact aacataatct taaactcttg aatatgtgcc ttctagaata     2040
   catattacaa gaaaactaca gggtccacac ggcaatcaga agaaaggagc tgagatgagg     2100
   ttttggaaaa ccctgacacc tttaaaaagc agtttttgaa agacaaaatt tagatttaat     2160
   ttacgtcttg agaaatacta tatatacaat atatattttg tgggcttaat tgaaacaaca     2220
   ttattttaaa atcaaagggg atatatgttt gtggaatgga ttttcctgaa gctgcttaac     2280
   agttgctttg gattctctaa gatgaatcca aatgtgaaag atgcatgtta ctgccaaaac     2340
   caaattgagc tcagcttcct aggcattacc caaaagcaag gtgtttaagt aattgccagc     2400
   ttttatacca tcatgagtgg tgacttaagg agaaatagct gtatagatga gtttttcatt     2460
   atttggaaat ttaggggtag aaaatgtttt cccctaattt tccagagaag cctattttta     2520
   tatttttaaa aaactgacag ggcccagtta aatatgattt gcatttttta aatttgccag     2580
   ttttattttc taaattcttt catgagcttg cctaaaattc ggaatggttt tcgggttgtg     2640
   gcaaacccca aagagagcac tgtccaagga tgtcgggagc atcctgctgc ttaggggaat 2700
   gttttcgcaa atgttgctct agtcagtcca gctcatctgc caaaatgtag ggctaccgtc 2760
   ttggatgcat gagctattgc tagagcatca tccttagaaa tcagtgcccc agatgtacat 2820
   gtgttgagcg tattcttgaa agtattgtgt ttatgcattt caatttcaat ggtgttggct 2880
   tcccctcccc accccacgcg tgcataaaaa ctggttctac aaatttttac ttgaagtacc 2940
   aggccgtttg ctttttcagg ttgttttgtt ttatagtatt aagtgaaatt ttaaatgcac 3000
   agttctattt gctatctgaa ctaattcatt tattaagtat atttgtaaaa gctaaggctc 3060
   gagttaaaac aatgaagtgt tttacaatga tttgtaaagg actatttata actaatatgg 3120
   ttttgttttc aatgaattaa gaaagattaa atatatcttt gtaaattatt ttatgtcata 3180
   gtttaattgg tctaccaagt aagacatctc aaatacagta gtataatgta tgaattttgt 3240
   aagtataaga aattttatta gacattctct tactttttgt aaatgctgta aatatttcat 3300
   aaattaacaa agtgtcactc cataaaaaga aagctaatac taatagccta aaagattttg 3360
   tgaaatttca tgaaaacttt ttaatggcaa taatgactaa agacctgctg taataaatgt 3420
   attaactgaa acctaaaaaa aaaaaaaaaa aa 3452
<210> 5
   <211> 3339
   <212> DNA
   <213> Mus musculus
<220>
   <221> source
   <222> 1..3339
   <223> /mol_type="unassigned DNA" /note="N° Accession NCBI : AF_073996" /organism="Mus musculus"
<400> 5
   cggtgagttc gctttcttgg ctgacctggc tcggagccgg gcattgcggg gatccaggat        60
   tggaaaggtt ccaggatggc ttctgcatca gcatctaagt ataattcaca ctccttggag       120
   aatgaatcca ttaagaaagt gtctcaagat ggagtcagtc aggatgtgag tgagactgtc       180
   cctcggctcc caggggagtt actaattact gaaaaagaag ttatttacat atgtcctttc       240
   aatggcccca ttaagggaag agtttacatc acaaattatc gtctttattt aagaagtttg       300
   gaaacggatt ctgctctaat acttgatgtt cctctgggtg tgatatcaag aattgaatat       360
   atgggaggcg cgactagtag aggagaaaat tcctatggtc tagatattac ttgtaaagat       420
   ttgagaaacc tgaggtttgc attgaagcaa gaaggccaca gcagaagaga tatgtttgag       480
   atccttgtaa aacatgcctt tcctctggca cacaatctgc cattatttgc atttgtaaat       540
   gaagagaagt ttaacgtgga tgggtggact gtttataatc cagttgaaga atatagaagg       600
   cagggcctgc ccaatcacca ttggaggata agttttatta acaagtgcta tgagctctgt       660
   gagacatacc ctgctctttt ggtggttccc tatcggacct cagatgatga tcttaggagg 720
   atcgcaacgt ttagatcccg aaatcggctt cctgtactgt cgtggattca cccagaaaac 780
   aaaatggtca ttatgcgctg cagtcagcct cttgtcggta tgagtggtaa aagaaataaa 840
   gatgacgaga aatacctgga tgtgatcagg gaaactaaca aacaaacttc taagctcatg 900
   atttatgatg cacgacccag tgtaaatgca gtcgccaaca aggcaacagg aggaggatat 960
   gaaagtgatg acgcatatca aaactcagaa ctttccttct tagacattca taatattcat 1020
   gttatgcgag aatctttaaa aaaagtgaaa gatattgttt atcccaacat agaagaatct 1080
   cattggttgt ccagtttgga gtctactcat tggttagaac atatcaagct tgttctgacc 1140
   ggtgccattc aagtggcaga ccaagtgtct tcaggaaaga gctcggtact tgtgcactgc 1200
   agtgacggat gggacaggac cgctcagctg acatccttgg ccatgctgat gttggacagc 1260
   ttctacagaa ctattgaagg ctttgagata ttggtacaga aagagtggat aagttttggc 1320
   cataaatttg catctagaat aggtcatggt gataaaaacc atgctgatgc tgatcgatct 1380
   cctatttttc ttcagtttat tgactgtgtg tggcagatgt cgaaacagtt ccccacagct 1440
   tttgagttca atgaaggctt tttgattacc gttttggatc atctgtatag ctgtcgattt 1500
   ggtactttct tattcaactg tgactcggct cgagaaagac agaaacttac agaaagaaca 1560
   gtttctctat ggtcgctaat taacagcaat aaagacaaat tcaaaaaccc cttctataca 1620
   aaagaaatca atcgggtttt gtatccagtt gccagcatgc gtcacttgga actgtgggtg 1680
   aattattaca tccgatggaa tcccagggtc aagcagcaac agcccaaccc agtggagcag 1740
   cgttacatgg agcttttggc cttgcgtgac gattatataa agaggctcga ggaattgcag 1800
   ctggccaact ccgccaagct tgctgatgcc cccgcttcga cttccagttc gtcacagatg 1860
   gtgccccatg tgcagacgca cttctgaggg gactcacttc tggcactgca cttgaactct 1920
   agataagtga aatagctgac tctcattctg ggcatgtgga caaagtagat ttaaagtgtc 1980
   tgcctccatt tagaagttca actaacatct tagacttttg agtatgtgcc ttctgtaata 2040
   catatcacaa gaaatcgatg gtgtccgtgt ggcaatcata aggaaggagt caagaggggg 2100
   ttctggaaaa tcctcatact tttttttaca aagcactttt gcaaagataa aacttaaatt 2160
   taatttacct ctatataaat tctacatata cagtatgtat tttgtgggct taattgaaat 2220
   attattttaa atccaggggg gagatttgtt tgcaaaatgt attttcctcc agctgcttat 2280
   aacagttgct ttggattatc taaaattaat ccaaatgtga aagatgggta ttactgccaa 2340
   agccaaattg cactctgctt cttcagcaaa ttccaagagc aaggcgttta aataattgcc 2400
   aatttttatt ttaccataag tggtaaggta aaaagaaaga tgaacatttc atcattttga 2460
   atttttgaaa ataaaaggtt ctcccatcat tttcaagagg aagcacattt ttatattaag 2520
   aaaaagtgat aaggtttgat ttttttttcc ctcaacattc tcagctttgc tttctaaatt     2580
   atcccatgat ttttgtctaa cactgagtca tactcaggtt gaaggaaacc cataaatagc     2640
   actgtgcgag gagctggctg gcttctgctg cttagaggaa tatgttcgca aacatgcctc     2700
   tagtcaattc gccttatctg ctgaagtgta ggggcaccgc cttgaatgga tgagctatgg     2760
   ctagagcatc tttctttaca gtaatgcccc aggtgtattc tgtttatgtc tctctgttta     2820
   aatggtgtgc gtgcataaaa acttgctctg cacattatta cttgaagtac tgggcaattt     2880
   gctttttcag gttttttttc attttgtttt gtagtatgaa atggaatttt aaatgcacag     2940
   ttctatttga tatccgaact aattcattta gtaaatatat ttgtaaaagc taaagttaaa     3000
   tcaattaatg ttttacagtg atttgtaaag gattatttat agctaatatg gttttgtttt     3060
   cagtgaatta agagagatta catttatctt tgtaaattat tttatgtcat agcttaatgg     3120
   cctaccaaat gagacatctc aaatataata gtataatgta tggattttgt aagtataaaa     3180
   attattagat attcgtttgc tttttgtaaa cactgtaaat atttcataaa ttaaaatgtg     3240
   tcactccata agaagaaaaa actaatacta atagttgaca ggaattggtg aaatttcatg     3300
   aaaatatttt cattgcaata aatattaaaa gacctgctg                            3339
<210> 6
   <211> 1841
   <212> DNA
   <213> Canis lupus
<220>
   <221> source
   <222> 1..1841
   <223> /mol_type="unassigned DNA" /note="N° Accession NCBI : XM_850116" /organism="Canis lupus"
<400> 6
   cggtggcgcc cggaccccga gtttccagta tggcttctgc accaacttct aaatataatt 60
   cacactcctt ggagaacgag tctattaaga ggacttctag ggatggagtt aattgggacc 120
   tgagtgaggc tgttcctcga cttccaggag aaactcgtat cactgacaaa gaagttattt 180
   acatatgtcc tttcaatggc cccattaagg gaagagttta catcacaaat tatcgtcttt 240
   atttaagaag tttggaaacg gattctgctc taatacttga tgttcctctg ggtgtgatct 300
   ccagaattga aaaaatggga ggcgcgacaa gtagaggaga aaattcgtat ggtctagata 360
   ttacttgtaa agacatgagg aacttgaggt tcgcgctgaa acaggaaggc cacagcagga 420
   gggatatgtt tgagatcctc acaagatacg cctttccctt ggcccacagt ctgccaatat 480
   ttgcatttct aaacgaagaa aagtttaacg tggatgggtg gacagtttat aatccagtcg 540
   aagaatacag aaggcagggc ttgcccaatc accactggag aataactttt atcaacaagt 600
   gctatgagct ctgtgacact tatcctgctc tcttggtggt tccatatcgt gcctcagatg 660
   acgatctcag gagagttgca acttttagat ccagaaatcg aattccagtg ctgtcatgga 720
   ttcatccaga aaacaagacg gtcattgtgc gctgcagcca gcctcttgtc ggaatgagtg 780
   gtaaacggaa taaagatgat gagaagtatc tcgatgttat cagggagact aacagacaaa 840
   tttctaaact cacaatctat gatgccagac ccaatgtaaa tgccgtggcc aacaaggcaa 900
   caggaggagg atatgaaagt gatgatgcat atcataacgc cgaacttttc ttcttagaca 960
   ttcataacat tcatgttatg cgggaatctt taaaaaaagt caaagacatc gtttatccta 1020
   atgtggaaga gtctcactgg ctgtccagtt tggagtctac ccattggtta gaacatatca 1080
   agcttgtttt gacgggagcc attcaagtag cagacagagt ttcttcaggg aagagctcag 1140
   tgctcgtgca ctgcagcgat ggatgggaca ggactgccca gctgacgtcc ttggccatgc 1200
   tgatgctcga cagcttctat cggagcatcg agggctttga aatattggta caaaaggaat 1260
   ggataagttt tggacataag tttgcatcta gaataggtca tggtgataaa aaccacgccg 1320
   acgctgaccg gtctcctatt tttctccagt ttattgattg tgtatggcaa atgtcaaaac 1380
   agttccctac agcttttgaa ttcaatgaac gatttttgat tacaattttg gatcatctgt 1440
   atagttgccg gtttggtacc ttcttgtaca actgtgaatc tgctcgggaa aaacagaaag 1500
   tgacggaacg aacagtatct ttatggtcac tgataaacag taataaggac aaattcaaaa 1560
   atcccttcta tactaaagaa atcaatcgag ttttatatcc agttgccagt atgcgtcact 1620
   tggaactttg ggtgaattac tacattagat ggaaccccag gatcaagcaa caacagccca 1680
   acccagtgga gcagcggtac gtggagctgt tggccttgcg tgacgaatac atacagcggc 1740
   tcgaggagct gcagctcgcc agctcggcca agctgcccga cccctcgacc tcaccggccg 1800
   ggccctcgca gatgatgccg cacgtgcgca cacacttctg a 1841
<210> 7
   <211> 821
   <212> PRT
   <213> Homo sapiens
<220>
   <223> N° Accession NCBI: NP_000061.1
<400> 7
<210> 8
   <211> 3316
   <212> DNA
   <213> Homo sapiens
<220>
   <221> source
   <222> 1..3316
   <223> /mol_type="unassigned DNA" /note="N° Accession NCBI: NM_000070 " /organism="Homo sapiens"
<400> 8
   cactctcttt ctctctccct ctggcatgca tgctgctggt aggagacccc caagtcaaca      60
   ttgcttcaga aatcctttag cactcatttc tcaggagaac ttatggcttc agaatcacag     120
   ctcggttttt aagatggaca taacctgtac gaccttctga tgggctttca actttgaact     180
   ggatgtggac acttttctct cagatgacag aattactcca acttcccctt tgcagttgct     240
   tcctttcctt gaaggtagct gtatcttatt ttctttaaaa agctttttct tccaaagcca     300
   cttgccatgc cgaccgtcat tagcgcatct gtggctccaa ggacagcggc tgagccccgg     360
   tccccagggc cagttcctca cccggcccag agcaaggcca ctgaggctgg gggtggaaac     420
   ccaagtggca tctattcagc catcatcagc cgcaattttc ctattatcgg agtgaaagag     480
   aagacattcg agcaacttca caagaaatgt ctagaaaaga aagttcttta tgtggaccct     540
   gagttcccac cggatgagac ctctctcttt tatagccaga agttccccat ccagttcgtc     600
   tggaagagac ctccggaaat ttgcgagaat ccccgattta tcattgatgg agccaacaga    660
   actgacatct gtcaaggaga gctaggggac tgctggtttc tcgcagccat tgcctgcctg    720
   accctgaacc agcaccttct tttccgagtc ataccccatg atcaaagttt catcgaaaac    780
   tacgcaggga tcttccactt ccagttctgg cgctatggag agtgggtgga cgtggttata    840
   gatgactgcc tgccaacgta caacaatcaa ctggttttca ccaagtccaa ccaccgcaat    900
   gagttctgga gtgctctgct ggagaaggct tatgctaagc tccatggttc ctacgaagct    960
   ctgaaaggtg ggaacaccac agaggccatg gaggacttca caggaggggt ggcagagttt   1020
   tttgagatca gggatgctcc tagtgacatg tacaagatca tgaagaaagc catcgagaga   1080
   ggctccctca tgggctgctc cattgatgat ggcacgaaca tgacctatgg aacctctcct   1140
   tctggtctga acatggggga gttgattgca cggatggtaa ggaatatgga taactcactg   1200
   ctccaggact cagacctcga ccccagaggc tcagatgaaa gaccgacccg gacaatcatt   1260
   ccggttcagt atgagacaag aatggcctgc gggctggtca gaggtcacgc ctactctgtc   1320
   acggggctgg atgaggtccc gttcaaaggt gagaaagtga agctggtgcg gctgcggaat   1380
   ccgtggggcc aggtggagtg gaacggttct tggagtgata gatggaagga ctggagcttt   1440
   gtggacaaag atgagaaggc ccgtctgcag caccaggtca ctgaggatgg agagttctgg   1500
   atgtcctatg aggatttcat ctaccatttc acaaagttgg agatctgcaa cctcacggcc   1560
   gatgctctgc agtctgacaa gcttcagacc tggacagtgt ctgtgaacga gggccgctgg   1620
   gtacggggtt gctctgccgg aggctgccgc aacttcccag atactttctg gaccaaccct   1680
   cagtaccgtc tgaagctcct ggaggaggac gatgaccctg atgactcgga ggtgatttgc   1740
   agcttcctgg tggccctgat gcagaagaac cggcggaagg accggaagct aggggccagt   1800
   ctcttcacca ttggcttcgc catctacgag gttcccaaag agatgcacgg gaacaagcag   1860
   cacctgcaga aggacttctt cctgtacaac gcctccaagg ccaggagcaa aacctacatc   1920
   aacatgcggg aggtgtccca gcgcttccgc ctgcctccca gcgagtacgt catcgtgccc   1980
   tccacctacg agccccacca ggagggggaa ttcatcctcc gggtcttctc tgaaaagagg   2040
   aacctctctg aggaagttga aaataccatc tccgtggatc ggccagtgaa aaagaaaaaa   2100
   accaagccca tcatcttcgt ttcggacaga gcaaacagca acaaggagct gggtgtggac   2160
   caggagtcag aggagggcaa aggcaaaaca agccctgata agcaaaagca gtccccacag   2220
   ccacagcctg gcagctctga tcaggaaagt gaggaacagc aacaattccg gaacattttc   2280
   aagcagatag caggagatga catggagatc tgtgcagatg agctcaagaa ggtccttaac   2340
   acagtcgtga acaaacacaa ggacctgaag acacacgggt tcacactgga gtcctgccgt   2400
   agcatgattg cgctcatgga tacagatggc tctggaaagc tcaacctgca ggagttccac   2460
   cacctctgga acaagattaa ggcctggcag aaaattttca aacactatga cacagaccag   2520
   tccggcacca tcaacagcta cgagatgcga aatgcagtca acgacgcagg attccacctc 2580
   aacaaccagc tctatgacat cattaccatg cggtacgcag acaaacacat gaacatcgac 2640
   tttgacagtt tcatctgctg cttcgttagg ctggagggca tgttcagagc ttttcatgca 2700
   tttgacaagg atggagatgg tatcatcaag ctcaacgttc tggagtggct gcagctcacc 2760
   atgtatgcct gaaccaggct ggcctcatcc aaagccatgc aggatcactc aggatttcag 2820
   tttcaccctc tatttccaaa gccatttacc tcaaaggacc cagcagctac acccctacag 2880
   gcttccaggc acctcatcag tcatgctcct cctccatttt accccctacc catccttgat 2940
   cggtcatgcc tagcctgacc ctttagtaaa gcaatgaggt aggaagaaca aacccttgtc 3000
   cctttgccat gtggaggaaa gtgcctgcct ctggtccgag ccgcctcggt tctgaagcga 3060
   gtgctcctgc ttaccttgct ctaggctgtc tgcagaagca cctgccggtg gcactcagca 3120
   cctccttgtg ctagagccct ccatcacctt cacgctgtcc caccatgggc caggaaccaa 3180
   accagcactg ggttctactg ctgtggggta aactaactca gtggaatagg gctggttact 3240
   ttgggctgtc caactcataa gtttggctgc attttgaaaa aagctgatct aaataaaggc 3300
   atgtgtatgg ctggtc 3316
<210> 9
   <211> 22
   <212> RNA
   <213> Homo sapiens
<220>
   <221> source
   <222> 1..22
   <223> /mol_type="other RNA" /note="N°Accession: MIMT0000241" /organism="Homo sapiens"
<400> 9
   auaagacgag caaaaagcuu gu 22
<210> 10
   <211> 22
   <212> DNA
   <213> Homo sapiens
<220>
   <221> source
   <222> 1..22
   <223> /mol_type="unassigned DNA" /organism="Homo sapiens"
<400> 10
   acaagctttt tgctcgtctt at 22
<210> 11
   <211> 1050
   <212> DNA
   <213> Homo sapiens
<220>
   <221> source
   <222> 1..1050
   <223> /mol_type="unassigned DNA" /note="Promoteur Desmine" /organism="Homo sapiens"
<400> 11
   cccccacagc tcctctcctg tgccttgttt cccagccatg cgttctcctc tataaatacc      60
   cgctctggta tttggggttg gcagctgttg ctgccaggga gatggttggg ttgacatgcg     120
   gctcctgaca aaacacaaac ccctggtgtg tgtgggcgtg ggtggtgtga gtagggggat     180
   gaatcaggga gggggcgggg gacccagggg gcaggagcca cacaaagtct gtgcgggggt     240
   gggagcgcac atagcaattg gaaactgaaa gcttatcaga ccctttctgg aaatcagccc     300
   actgtttata aacttgaggc cccaccctcg acagtaccgg ggaggaagag ggcctgcact     360
   agtccagagg gaaactgagg ctcagggcca gctcgcccat agacatacat ggcaggcagg     420
   ctttggccag gatccctccg cctgccaggc gtctccctgc cctcccttcc tgcctagaga     480
   cccccaccct caagcctggc tggtctttgc ctgagaccca aacctcttcg acttcaagag     540
   aatatttagg aacaaggtgg tttagggcct ttcctgggaa caggccttga ccctttaaga     600
   aatgacccaa agtctctcct tgaccaaaaa ggggaccctc aaactaaagg gaagcctctc     660
   ttctgctgtc tcccctgacc ccactccccc ccaccccagg acgaggagat aaccagggct     720
   gaaagaggcc cgcctggggg ctgcagacat gcttgctgcc tgccctggcg aaggattggt     780
   aggcttgccc gtcacaggac ccccgctggc tgactcaggg gcgcaggcct cttgcggggg     840
   agctggcctc cccgccccca cggccacggg ccgccctttc ctggcaggac agcgggatct     900
   tgcagctgtc aggggagggg aggcgggggc tgatgtcagg agggatacaa atagtgccga     960
   cggctggggg ccctgtctcc cctcgccgca tccactctcc ggccggccgc ctgcccgccg    1020
   cctcctccgt gcgcccgcca gcctcgcccg                                     1050
<210> 12
   <211> 1654
   <212> DNA
   <213> Homo sapiens
<220>
   <221> source
   <222> 1..1654
   <223> /mol_type="unassigned DNA" /note="Promoteur calpaine 3" /organism="Homo sapiens"
<400> 12
   cacatgcctc cactctgcca tacttgaaat gtgctcatct ccttacagcc cagggagcag      60
   ctattgtggg tagaagacaa ggtggaggcc aggcaggcac ttcccttccc cagagccact 120
   tatgctctca tctaagagcc ctgaaaccag gtgtgacatc ccaggagttg acagacagtc 180
   tggttcagta tctaattcca acttctgtct cagatgccta atgtggcatg gctgaatgag 240
   tcaacatata acctgtacag taagtcctca cttaacatca ttgataggtg cttgtaaact 300
   gtgactttaa cgaaaacata ccgtgtgctg tagggactta actcttgttt atatcagtta 360
   gcctggtttc actatacagt acatcatttt gcttaaagtc acagcttacg agaacctatc 420
   gatgatgtta agtgaggatt ttctctgctc aggtgcactt tttttttttt tttaagacgg 480
   agtctctttc tgtcacctgg gctggagtgc agtggcgcga tctgggttca ctacaacctc 540
   tgcctcctgg gttcaagcaa ttcttctgtc tcagcctccc aagtagctgg gattacaggc 600
   acccgccgcc acacccggct tatttttgta tttttagtag agacagggtt tcactattgt 660
   tggccatgct ggtctcgaac tcctgacctc atgtgatcca cccgcctcgg cctcccaaag 720
   tgcagagatt agagacgtga gccacatggc ccagcaggac cactttttag cagattcagt 780
   cccagtgttc attttgtgga tggggagaga caagaggtgg caaggtcaag tgtgcaggta 840
   gagacaggga ttttctcaaa tgaggactct gctgagtagc attttccatg cagacatttc 900
   caatgagcgc tgacccaaga acattctaaa aaagatacca aatctaacat tgaataatgt 960
   tctgatatcc taaaatttta ggactaaaaa tcatgttctc taaaattcac agaatatttt 1020
   tgtagaattc agtacctccc gttcacccta actagctttt ttgcaatatt gttttccatt 1080
   catttgatgg ccagtagttg ggtggtctgt ataactgcct actcaataac atgtcagcag 1140
   ttctcagctt ctttccagtg ttcaccttac tcagatactc ccttttcatt ttctggcaac 1200
   accagcactt catggcaaca gaaatgtccc tagccaggtt ctctctctac catgcagtct 1260
   ctcttgctct catactcaca gtgtttcttc acatctattt ttagttttcc tggctcaagc 1320
   atcttcaggc cactgaaaca caaccctcac tctctttctc tctccctctg gcatgcatgc 1380
   tgctggtagg agacccccaa gtcaacattg cttcagaaat cctttagcac tcatttctca 1440
   ggagaactta tggcttcaga atcacagctc ggtttttaag atggacataa cctgtacgac 1500
   cttctgatgg gctttcaact ttgaactgga tgtggacact tttctctcag atgacagaat 1560
   tactccaact tcccctttgc agttgcttcc tttccttgaa ggtagctgta tcttattttc 1620
   tttaaaaagc tttttcttcc aaagccactt gcca 1654
<210> 13
   <211> 805
   <212> DNA
   <213> Homo sapiens
<220>
   <221> source
   <222> 1..805
   <223> /mol_type="unassigned DNA" /note="promoteur miARN206" /organism="Homo sapiens"
<400> 13
   gggggccaac tcttcctttg gcatatgttt ccccattttc tggcagagaa tcagatacca      60
   caaagttcaa aaccccatct ccctccagcc agggtggcca tccagaccct gagtggctca     120
   acagctgcca atgtccctca tccttctgag gctcaggcct cacagattgt ggggcaggtg     180
   atgggctagg gggagcagaa gcccgacaaa aggatccttc ccacagtgaa caatggtgct     240
   tggaatgctg gatgggcagc tgctgcccat caacaagcac ccaaaacaga tagacgtaca     300
   gtaggaagta caggagggcc ggtgtgtttc taagcatgag tggctctctg cgtgaatgtg     360
   gaaaatttct ctgttggatt ctctcttctt tttaattttc ccttcactgg atcccaaaca     420
   ttaaaaaaga atcacattca aaatgcacaa aaacagcagc agtgaattaa ttagtagtaa     480
   taacaaagga ctggatagac tgtagctgca caagaataag ccagggaaac gtggtgctgc     540
   ttatctgtga acaaacagta ggaaggattt ggtcccaagc agcactgcca ttcctcacaa     600
   cagatttatt tcagcatgat ttggtcgggc gggggggatt taggatgagt tgagatccca     660
   gtgatcttct cgctaagagt ttcctgcctg ggcaaggagg aaagatgcta caagtggccc     720
   acttctgaga tgcgggctgc ttctggatga cactgcttcc cgaggccaca tgcttcttta     780
   tatccccata tggattactt tgcta                                           805
<210> 14
   <211> 1812
   <212> DNA
   <213> Mus musculus
<220>
   <221> source
   <222> 1..1812
   <223> /mol_type="unassigned DNA" /note="ORF MTM1" /organism="Mus musculus"
<400> 14
   atggcttctg catcagcatc taagtataat tcacactcct tggagaatga atccattaag      60
   aaagtgtctc aagatggagt cagtcaggat gtgagtgaga ctgtccctcg gctcccaggg     120
   gagttactaa ttactgaaaa agaagttatt tacatatgtc ctttcaatgg ccccattaag     180
   ggaagagttt acatcacaaa ttatcgtctt tatttaagaa gtttggaaac ggattctgct     240
   ctaatacttg atgttcctct gggtgtgata tcaagaattg aaaaaatggg aggcgcgaca     300
   agtagaggag aaaattccta tggtctagat attacttgta aagatttgag aaacctgagg     360
   tttgcattga agcaagaagg ccacagcaga agagatatgt ttgagatcct tgtaaaacat     420
   gcctttcctc tggcacacaa tctgccatta tttgcatttg taaatgaaga gaagtttaac     480
   gtggatgggt ggactgttta taatccagtt gaagaatata gaaggcaggg cctgcccaat      540
   caccattgga ggataagttt tattaacaag tgctatgagc tctgtgagac ataccctgct      600
   cttttggtgg ttccctatcg gacctcagat gatgatctta ggaggatcgc aacgtttaga      660
   tcccgaaatc ggcttcctgt actgtcgtgg attcacccag aaaacaaaat ggtcattatg      720
   cgctgcagtc agcctcttgt cggtatgagt ggtaaaagaa ataaagatga cgagaaatac      780
   ctggatgtga tcagggaaac taacaaacaa acttctaagc tcatgattta tgatgcacga      840
   cccagtgtaa atgcagtcgc caacaaggca acaggaggag gatatgaaag tgatgacgca      900
   tatcaaaact cagaactttc cttcttagac attcataata ttcatgttat gcgagaatct      960
   ttaaaaaaag tgaaagatat tgtttatccc aacatagaag aatctcattg gttgtccagt     1020
   ttggagtcta ctcattggtt agaacatatc aagcttgttc tgaccggtgc cattcaagtg     1080
   gcagaccaag tgtcttcagg aaagagctcg gtacttgtgc actgcagtga cggatgggac     1140
   aggaccgctc agctgacatc cttggccatg ctgatgttgg acagcttcta cagaactatt     1200
   gaaggctttg agatattggt acagaaagag tggataagtt ttggccataa atttgcatct     1260
   agaataggtc atggtgataa aaaccatgct gatgctgatc gatctcctat ttttcttcag      1320
   tttattgact gtgtgtggca gatgtcgaaa cagttcccca cagcttttga gttcaatgaa     1380
   ggctttttga ttaccgtttt ggatcatctg tatagctgtc gatttggtac tttcttattc     1440
   aactgtgact cggctcgaga aagacagaaa cttacagaaa gaacagtttc tctatggtcg     1500
   ctaattaaca gcaataaaga caaattcaaa aaccccttct atacaaaaga aatcaatcgg      1560
   gttttgtatc cagttgccag catgcgtcac ttggaactgt gggtgaatta ttacatccga     1620
   tggaatccca gggtcaagca gcaacagccc aacccagtgg agcagcgtta catggagctt     1680
   ttggccttgc gtgacgatta tataaagagg ctcgaggaat tgcagctggc caactccgcc     1740
   aagcttgctg atgcccccgc ttcgacttcc agttcgtcac agatggtgcc ccatgtgcag      1800
   acgcacttct ga                                                         1812
<210> 15
   <211> 22
   <212> DNA
   <213> Artificial Sequence
<220>
   <221> source
   <222> 1..22
   <223> /mol_type="unassigned DNA" /note="AAV forward" /organism="Artificial Sequence"
<400> 15
   ctccatcact aggggttcct tg 22
<210> 16
   <211> 18
   <212> DNA
   <213> Artificial Sequence
<220>
   <221> source
   <222> 1..18
   <223> /mol_type="unassigned DNA" /note="AAV reverse" /organism="Artificial Sequence"
<400> 16
   gtagataagt agcatggc 18
<210> 17
   <211> 17
   <212> DNA
   <213> Artificial Sequence
<220>
   <221> source
   <222> 1..17
   <223> /mol_type="unassigned DNA" /note="AAV probe" /organism="Artificial Sequence"
<400> 17
   tagttaatga ttaaccc 17
<210> 18
   <211> 21
   <212> DNA
   <213> Artificial Sequence
<220>
   <221> source
   <222> 1..21
   <223> /mol_type="unassigned DNA" /note="Titine forward" /organism="Artificial Sequence"
<400> 18
   aaaacgagca gtgacgtgag c 21
<210> 19
   <211> 20
   <212> DNA
   <213> Artificial Sequence
<220>
   <221> source
   <222> 1..20
   <223> /mol_type="unassigned DNA" /note="Titine reverse" /organism="Artificial Sequence"
<400> 19
   ttcagtcatg ctgctagcgc 20
<210> 20
   <211> 25
   <212> DNA
   <213> Artificial Sequence
<220>
   <221> source
   <222> 1..25
   <223> /mol_type="unassigned DNA" /note="Ttine probe" /organism="Artificial Sequence"
<400> 20
   tgcacggaag cgtctcgtct cagtc 25
<210> 21
   <211> 16
   <212> DNA
   <213> Artificial Sequence
<220>
   <221> source
   <222> 1..16
   <223> /mol_type="unassigned DNA" /note="CAPN3sfr.f " /organism="Artificial Sequence"
<400> 21
   cgcctccaag gcccgt 16
<210> 22
   <211> 16
   <212> DNA
   <213> Artificial Sequence
<220>
   <221> source
   <222> 1..16
   <223> /mol_type="unassigned DNA" /note="CAPN3sfr.r " /organism="Artificial Sequence"
<400> 22
   ggcggaagcg ctggct 16
<210> 23
   <211> 21
   <212> DNA
   <213> Artificial Sequence
<220>
   <221> source
   <222> 1..21
   <223> /mol_type="unassigned DNA" /note="MGBTUCAPN3.p " /organism="Artificial Sequence"
<400> 23
   ctacatcaac atgagagagg t 21
<210> 24
   <211> 16
   <212> DNA
   <213> Artificial Sequence
<220>
   <221> source
   <222> 1..16
   <223> /mol_type="unassigned DNA" /note="CAPN3.f " /organism="Artificial Sequence"
<400> 24
   cgcctccaag gccagg 16
<210> 25
   <211> 16
   <212> DNA
   <213> Artificial Sequence
<220>
   <221> source
   <222> 1..16
   <223> /mol_type="unassigned DNA" /note="CAPN3.r " /organism="Artificial Sequence"
<400> 25
   ggcggaagcg ctggga 16
<210> 26
   <211> 20
   <212> DNA
   <213> Artificial Sequence
<220>
   <221> source
   <222> 1..20
   <223> /mol_type="unassigned DNA" /note="CAPN3.p " /organism="Artificial Sequence"
<400> 26
   tacatcaaca tgcgggaggt 20
<210> 27
   <211> 20
   <212> DNA
   <213> Artificial Sequence
<220>
   <221> source
   <222> 1..20
   <223> /mol_type="unassigned DNA" /note="MH181PO.F " /organism="Artificial Sequence"
<400> 27
   ctccaagcag atgcagcaga 20
<210> 28
   <211> 20
   <212> DNA
   <213> Artificial Sequence
<220>
   <221> source
   <222> 1..20
   <223> /mol_type="unassigned DNA" /note="M267PO.R " /organism="Artificial Sequence"
<400> 28
   accatgatgc gcaaggctat 20
<210> 29
   <211> 22
   <212> DNA
   <213> Artificial Sequence
<220>
   <221> source
   <222> 1..22
   <223> /mol_type="unassigned DNA" /note="M225PO.p " /organism="Artificial Sequence"
<400> 29
   ccgtggtgct gatgggcaag aa 22